(19)

**Europäisches Patentamt**
**European Patent Office**
**Office européen des brevets**

(11) **EP 4 559 383 A1**

(12) **EUROPEAN PATENT APPLICATION**
published in accordance with Art. 153(4) EPC

(43) Date of publication:
28.05.2025 Bulletin 2025/22

(51) International Patent Classification (IPC):
*A61B 5/024* (2006.01)

(21) Application number: 23878916.8

(22) Date of filing: 22.09.2023

(86) International application number:
PCT/CN2023/120880

(87) International publication number:
WO 2024/082923 (25.04.2024 Gazette 2024/17)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 19.10.2022 CN 202211282519

(71) Applicant: Honor Device Co., Ltd.
Shenzhen, Guangdong 518040 (CN)

(72) Inventors:
• MA, Ruiqing
Shenzhen, Guangdong 518040 (CN)
• LI, Danhong
Shenzhen, Guangdong 518040 (CN)
• LU, Chenxi
Shenzhen, Guangdong 518040 (CN)
• ZHANG, Xiaowu
Shenzhen, Guangdong 518040 (CN)

(74) Representative: Thun, Clemens
Mitscherlich PartmbB
Patent- und Rechtsanwälte
Karlstraße 7
80333 München (DE)

(54) **ARRHYTHMIA RECOGNITION METHOD AND WEARABLE DEVICE**

(57) This application provides a heart rhythm abnormality identification method and a wearable device. The method includes: The wearable device obtains heart rate data of a user; and determines a first feature and a second feature of the heart rate data based on the heart rate data and a Poincare plot. The first feature is a standard deviation of a distance from an origin of the Poincare plot to a first straight line, the first straight line is a straight line formed by first heart rate data located in an area 2 in the Poincare plot and second heart rate data located in an area 4 in the Poincare plot, the second feature is a standard deviation of a distance from the origin of the Poincare plot to a second straight line, and the second straight line is a straight line formed by third heart rate data located in the area 4 in the Poincare plot and fourth heart rate data located in the area 2 in the Poincare plot. The wearable device inputs the target feature to an identification model, to obtain an identification result. The identification model is configured to identify, based on the input, whether a heart rhythm is abnormal. This helps improve accuracy of heart rhythm abnormality identification.

FIG. 2

## Description

This application claims priority to Chinese Patent Application No. 202211282519.4, filed with the China National Intellectual Property Administration on October 19, 2022 and entitled "HEART RHYTHM ABNORMALITY IDENTIFICATION METHOD AND WEARABLE DEVICE", which is incorporated herein by reference in its entirety.

## TECHNICAL FIELD

This application relates to the field of terminal technologies, and in particular, to a heart rhythm abnormality identification method and a wearable device.

## BACKGROUND

With the development of wearable devices, a wearable device can support more functions. Currently, the wearable device can detect a heart rhythm of a user, to determine whether the heart rhythm of the user is normal or abnormal.

Currently, the wearable device may calculate an average heart rate of the user based on a photoplethysmography (photoplethysmography, PPG) signal, and identify, based on the average heart rate, whether the heart rhythm of the user is normal or abnormal.

However, misidentification often occurs on the wearable device, resulting in low identification accuracy. For example, the wearable device mistakenly identifies a normal heart rhythm as an abnormal heart rhythm (such as premature beat), or the wearable device mistakenly identifies an abnormal heart rhythm (such as atrial fibrillation) as a normal heart rhythm.

## SUMMARY

This application provides a heart rhythm abnormality identification method and a wearable device, applied to the field of terminal technologies, to help improve accuracy of heart rhythm abnormality identification.

According to a first aspect, this application provides a heart rhythm abnormality identification method. The method includes: obtaining heart rate data of a user; determining a target feature of the heart rate data based on the heart rate data and a Poincare plot, where the target feature includes a first feature and a second feature, the first feature is a standard deviation of a distance from an origin of the Poincare plot to a first straight line, the first straight line is a straight line formed by first heart rate data located in an area 2 in the Poincare plot and second heart rate data located in an area 4 in the Poincare plot, an obtaining time of the first heart rate data is earlier than an obtaining time of the second heart rate data, the obtaining time of the first heart rate data is adjacent to the obtaining time of the second heart rate data, the second feature is a standard deviation of a distance from the origin of the Poincare plot to a second straight line, the second straight line is a straight line formed by third heart rate data located in the area 4 in the Poincare plot and fourth heart rate data located in the area 2 in the Poincare plot, an obtaining time of the third heart rate data is earlier than an obtaining time of the fourth heart rate data, and the obtaining time of the third heart rate data is adjacent to the obtaining time of the fourth heart rate data; and inputting the target feature to an identification model, to obtain an identification result, where the identification model is configured to identify, based on the input, whether a heart rhythm is abnormal.

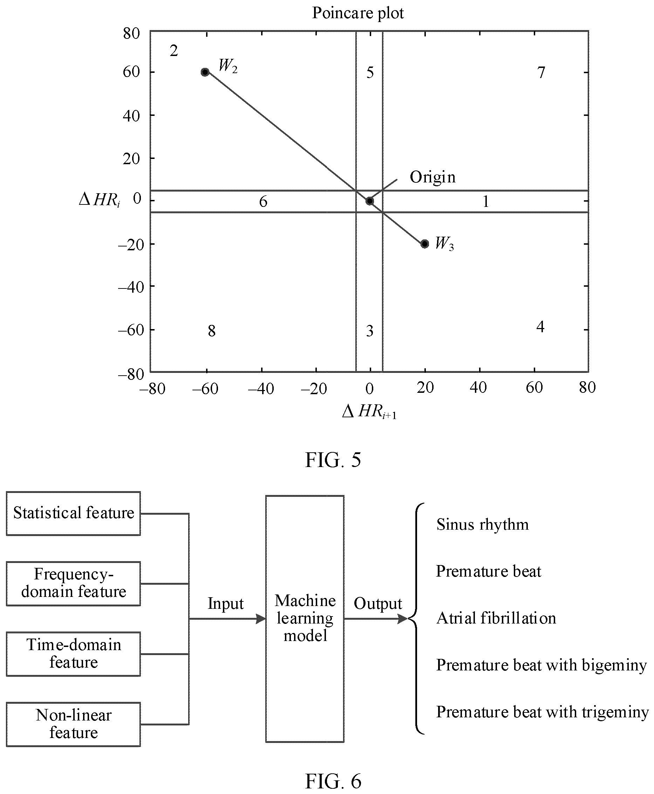

The heart rate data of the user is heart rate data of the user within a continuous period of time. The heart rate data may be represented in a form of a set or an array. This is not limited in this application. The heart rate data of the user may be obtained based on a PPG signal of the user, but this application is not limited thereto. If the heart rate data of the user is obtained based on a PPG signal of the user, a specific implementation may be that a wearable device obtains peak value data based on the PPG signal of the user, then obtains inter-beat interval (inter-beat interval, IBI) data based on the peak value data, and then obtains the heart rate data based on the IBI data. For a specific calculation method, refer to S209 to S211 shown in FIG. 5 in embodiments.

If the heart rate data of the user is represented in a form of a set, the heart rate data may be referred to as a heart rate set, and may be represented by a symbol HR. If the heart rate set includes q-1 elements, $\mathbf{HR}=[HR_1, HR_2, ..., HR_{q-1}]$. The Poincare plot uses an origin (0, 0) as a center, whose upper, lower, left, and right sides are bounded by a maximum value of heart rate differences; or uses an origin as a center, whose upper, lower, left, and right sides are not bounded.

The wearable device may calculate differences between adjacent heart rate data based on the heart rate data, determine an area in the Poincare plot based on difference data, and extract the target feature.

For example, for the heart rate set $\mathbf{HR}=[HR_1, HR_2, ..., HR_{q-1}]$, the heart rate differences may be represented by a difference set. The difference set may be represented by $\Delta\mathbf{HR}$, and $\Delta\mathbf{HR}=[\Delta HR_1, \Delta HR_2, ..., \Delta HR_{q-2}]$. $\Delta HR_1=HR_2-HR_1$, $\Delta HR_2=HR_3-HR_2$, ..., and $\Delta HR_{q-2}=HR_{q-1}-HR_{q-2}$. A horizontal coordinate of the Poincare plot may be represented by $\Delta HR_{i+1}$, and a vertical coordinate may be represented by $\Delta HR_i$. The wearable device may construct coordinates based on adjacent elements (for example, $\Delta HR_1$ and $\Delta HR_2$, and $\Delta HR_{q-3}$ and $\Delta HR_{q-2}$) in the difference set $\Delta \mathbf{HR}$, to obtain a coordinate set. If the coordinate set may be represented by $\mathbf{W}$, $\mathbf{W}=[W_1, W_2, ..., W_{q-3}]$. $W_1=(\Delta HR_2, \Delta HR_1)$, $W_2=(\Delta HR_3, \Delta HR_2)$, ..., and $W_{q-3}=(\Delta HR_{q-2}, \Delta HR_{q-3})$. The Poincare plot may include nine areas: an area 0, an area 1, an area 2, an area 3, an area 4, an area 5, an area 6, an area 7, and an area 8. A range of the area 0 may be $(-5 \leq HR_{i+1} \leq 5, -5 \leq HR_i \leq 5)$, a range of the area 1 may be $(5 < HR_{i+1}, -5 \leq HR_i \leq 5)$, a range of the area 2 may be $(HR_{i+1}, < -5, 5 < HR_i)$, a range of the area 3 may be $(-5 \leq HR_{i+1} \leq 5, Hg_i < -5)$, a range of the area 4 may be $(5 < HR_{i+1}, HR_i < -5)$, a range of the area 5 may be $(-5 \leq HR_{i+1} \leq 5, 5 < HR_i)$, a range of the area 6 may be $(HR_{i+1} < -5, -5 \leq HR_i \leq 5)$, and a range of the area 7 may be $(5 < HR_{i+1}, 5 < HR_i)$, and a range of the area 8 may be $(HR_{i+1} < -5, HR_i < -5)$. The wearable device may determine, based on coordinate points in the coordinate set $\mathbf{W}$ and the ranges of the nine areas, areas of the coordinate points in the Poincare plot, and extract the target feature.

If coordinate points formed by adjacent differences in the difference data may be represented by the coordinate set $\mathbf{W}$, the first heart rate data, the second heart rate data, the third heart rate data, and the fourth heart rate data are all elements in the coordinate set $\mathbf{W}$. If the first heart rate data and the fourth heart rate data are located in the area 2 in the Poincare plot, the first heart rate data and the fourth heart rate data may both be referred to as elements located in the area 2 in the coordinate set $\mathbf{W}$. If the second heart rate data and the third heart rate data are located in the area 4 in the Poincare plot, the second heart rate data and the third heart rate data may both be referred to as elements located in the area 4 in the coordinate set $\mathbf{W}$.

That an obtaining time of the first heart rate data is earlier than an obtaining time of the second heart rate data, and the obtaining time of the first heart rate data is adjacent to the obtaining time of the second heart rate data means that a location of the first heart rate data in the coordinate set $\mathbf{W}$ is in front of a location of the second heart rate data in the coordinate set $\mathbf{W}$, and the location of the first heart rate data in the coordinate set $\mathbf{W}$ is adjacent to the location of the second heart rate data in the coordinate set $\mathbf{W}$. That an obtaining time of the third heart rate data is earlier than an obtaining time of the fourth heart rate data, and the obtaining time of the third heart rate data is adjacent to the obtaining time of the second heart rate data means that a location of the first heart rate data in the coordinate set $\mathbf{W}$ is in front of a location of the second heart rate data in the coordinate set $\mathbf{W}$, and the location of the first heart rate data in the coordinate set $\mathbf{W}$ is adjacent to the location of the second heart rate data in the coordinate set $\mathbf{W}$.

The first feature is the standard deviation of the distance from the origin (0, 0) of the Poincare plot to the first straight line, and the second feature is the standard deviation of the distance from the origin of the Poincare plot to the second straight line. The first feature in embodiments is a feature 6 ($sd\_d_{24}$), and the second feature in embodiments is a feature 9 ($sd\_d_{42}$).

The terminal device may input the first feature and the second feature to an identification model, to obtain an identification result. The identification model is configured to identify, based on the input, whether a heart rhythm is abnormal.

The identification model may also be referred to as a machine learning model. This is not limited in embodiments of this application. The identification result has a plurality of implementations. In a possible implementation, the identification result may be that the heart rhythm is normal or the heart rhythm is abnormal. In another possible implementation, the identification result may be that the heart rhythm is normal or a type of heart rhythm abnormality.

According to the heart rhythm abnormality identification method provided in this application, a target feature is extracted based on distribution of heart rate data in a Poincare plot, and the target feature is input to a machine learning model, to obtain an identification result. Compared with using an average heart rate to represent an abnormal heart rhythm, using the target feature to represent an abnormal heart rhythm helps improve accuracy of heart rhythm abnormality identification. Compared with a method of determining based on a threshold, using the machine learning model to identify an abnormal heart rhythm helps improve accuracy and flexibility of identification.

With reference to the first aspect, in some implementations of the first aspect, the target feature further includes a third feature and a fourth feature, the third feature is a modulus of an angle corresponding to the first straight line, and the fourth feature is a modulus of an angle corresponding to the second straight line.

The first straight line is the straight line formed by the first heart rate data located in the area 2 in the Poincare plot and the second heart rate data located in the area 4 in the Poincare plot. The wearable device may calculate a slope of the first straight line based on the first heart rate data and the second heart rate data, then determine, based on the slope of the first straight line, the angle corresponding to the first straight line, and finally obtain the third feature. Similarly, the second straight line is the straight line formed by the third heart rate data located in the area 4 in the Poincare plot and the fourth heart rate data located in the area 2 in the Poincare plot. The wearable device may calculate a slope of the second straight line based on the third heart rate data and the fourth heart rate data, then determine, based on the slope of the second straight line, the angle corresponding to the second straight line, and finally obtain the fourth feature.

The third feature in embodiments is a feature 4 ($\theta_{24}$), and the fourth feature in embodiments is a feature 7 ($\theta_{42}$).

According to the heart rhythm abnormality identification method provided in this application, more features are extracted based on the first straight line and the second straight line, to obtain the third feature and the fourth feature, so that a distribution feature of the heart rate data can be more represented by using the first feature, the second feature, the third feature, and the fourth feature, which helps improve accuracy of heart rhythm abnormality identification.

With reference to the first aspect, in some implementations of the first aspect, the target feature further includes a fifth feature and a sixth feature, the fifth feature is a standard deviation of the angle corresponding to the first straight line, and the sixth feature is a standard deviation of the angle corresponding to the second straight line.

The heart rate data may include a plurality of pieces of first heart rate data and second heart rate data meeting the condition. The wearable device may calculate slopes of a plurality of first straight lines based on the plurality of pieces of first heart rate data and second heart rate data meeting the condition, determine, based on the slopes of the plurality of first straight lines, angles corresponding to the plurality of first straight lines, and then calculate a standard deviation based on the plurality of angles, to obtain the fifth feature. Similarly, the heart rate data may include a plurality of pieces of third heart rate data and fourth heart rate data meeting the condition. The wearable device may calculate slopes of a plurality of second straight lines based on the plurality of pieces of third heart rate data and fourth heart rate data meeting the condition, determine, based on the slopes of the plurality of second straight lines, angles corresponding to the plurality of second straight lines, and then calculate a standard deviation based on the plurality of angles, to obtain the sixth feature.

The fifth feature in embodiments is a feature 5 ($sd_{24}$), and the sixth feature in embodiments is a feature 8 ($sd_{42}$).

According to the heart rhythm abnormality identification method provided in this application, more features are extracted based on the first straight lines and the second straight lines, to obtain the fifth feature and the sixth feature, so that a distribution feature of the heart rate data can be more represented by using the first feature, the second feature, the fifth feature, and the sixth feature (or the first feature, the second feature, the third feature, the fourth feature, the fifth feature, and the sixth feature), which helps improve accuracy of heart rhythm abnormality identification.

With reference to the first aspect, in some implementations of the first aspect, the target feature further includes a seventh feature, an eighth feature, and a ninth feature, the seventh feature is a ratio of a quantity of data pieces of the heart rate data that are located in an area 0 in the Poincare plot to a total quantity of pieces of the heart rate data, the eighth feature is a standard deviation of an angle that uses heart rate data located in the area 2 as a vertex in three pieces of adjacent heart rate data of the heart rate data that are respectively located in an area 1, the area 2, and an area 3 in the Poincare plot, and the ninth feature is a standard deviation of an angle that uses heart rate data located in the area 4 as a vertex in three pieces of adjacent heart rate data of the heart rate data that are respectively located in an area 6, the area 4, and an area 5 in the Poincare plot.

That the seventh feature is a ratio of a quantity of data pieces of the heart rate data that are located in an area 0 in the Poincare plot to a total quantity of pieces of the heart rate data means that a ratio of a quantity of elements located in the area 0 to a total quantity of elements in the coordinate set W. The seventh feature in embodiments is referred to as a feature 1 ($r_{zero}$).

The eighth feature is the standard deviation of the angle that uses the heart rate data located in the area 2 as the vertex in the three pieces of adjacent heart rate data of the heart rate data that are respectively located in the area 1, the area 2, and the area 3 in the Poincare plot. Specifically, the wearable device may construct a triangle by using three adjacent elements in the coordinate set W that are respectively located in the area 1, the area 2, and the area 3, and calculate, based on a cosine theorem, an angle that uses the element located in the area 2 as a vertex. If there are a plurality of such angles in the coordinate set W, the wearable device may calculate a standard deviation of these angles to obtain the eighth feature. The seventh feature in embodiments is referred to as a feature 2 ($sd_{123}$).

The ninth feature is the standard deviation of the angle that uses the heart rate data located in the area 4 as the vertex in the three pieces of adjacent heart rate data of the heart rate data that are respectively located in the area 6, the area 4, and the area 5 in the Poincare plot. Specifically, the wearable device may construct a triangle by using three adjacent elements in the coordinate set **W** that are respectively located in the area 6, the area 4, and the area 5, and calculate, based on the cosine theorem, an angle that uses the element located in the area 4 as a vertex. If there are a plurality of such angles in the coordinate set **W,** the wearable device may calculate a standard deviation of these angles to obtain the ninth feature. The ninth feature in embodiments is referred to as a feature 3 ($sd_{645}$).

According to the heart rhythm abnormality identification method provided in this application, more features are extracted based on distribution of the heart rate data in the Poincare plot, to obtain the seventh feature, the eighth feature, and the ninth feature, which further helps represent a distribution feature of the heart rate data, and helps improve accuracy of heart rhythm abnormality identification.

With reference to the first aspect, in some implementations of the first aspect, the heart rate data is obtained based on inter-beat interval IBI data of a photoplethysmography PPG signal, and the target feature further includes at least one of the following: a ratio of a quantity of differences greater than 50 milliseconds between adjacent pieces of data in the IBI data to a total quantity of pieces in the IBI data, a mean value of the IBI data, a median of the IBI data, a standard deviation of the IBI data, a root mean square of differences between adjacent pieces of data in the IBI data, or a standard deviation of the differences between the adjacent pieces of data in the IBI data.

The IBI data may be represented in a form of a set or a numerical value. This is not limited in embodiments of this application. If the IBI data is represented in a form of a set, the IBI data may be referred to as an IBI set. The IBI set may be denoted as $\mathbf{TP}=[TP_1, TP_2, ..., TP_{q-1}]$. The wearable device may extract a time-domain feature in a time-domain dimension based on elements in the IBI set ($\mathbf{TP}=[TP_1, TP_2, ..., TP_{q-1}]$), and determine the time-domain feature as the target feature.

The time-domain feature may include a ratio (which may be represented by a symbol pNN50) of a quantity of differences greater than 50 milliseconds between adjacent pieces of data in the IBI data to a total quantity of pieces in the IBI data, a mean value (which may be represented by a symbol IBI_mean) of the IBI data, a median (which may be represented by a symbol IBI_median) of the IBI data, a standard deviation (which may be represented by a symbol SDNN) of the IBI data, a root mean square (which may be represented by a symbol RMSSD) of differences between adjacent pieces of data in the IBI data, or a standard deviation (which may be represented by a symbol SDSD) of the differences between the adjacent pieces of data in the IBI data.

In an example, the time-domain feature may include IBI_mean, SDNN, and SDSD.

In another example, the time-domain feature may include IBI_mean, IBI_median, SDNN, RMSSD, SDSD, and pNN50.

According to the heart rhythm abnormality identification method provided in this application, the target feature includes the time-domain feature and the distribution feature of the heart rate data in the Poincare plot, so that features are extracted in a plurality of dimensions, which further helps represent a distribution feature of the heart rate data, and helps improve accuracy of heart rhythm abnormality identification.

With reference to the first aspect, in some implementations of the first aspect, the heart rate data is obtained based on the IBI data of the PPG signal; and the method further includes: generating a power spectrum chart based on the IBI data; and determining at least one of a ratio of low frequency power to high frequency power, ultra low frequency power, the low frequency power, or the high frequency power in the power spectrum chart as the target feature.

The wearable device may perform a fast fourier transform (fast fourier transform, FFT) or an autoregressive (autoregressive, AR) parameter modeling operation on the IBI data to obtain a power spectrum chart that uses a frequency (whose unit may be Hertz) as a horizontal coordinate and a power spectrum density as a vertical coordinate, extract a frequency-domain feature based on the power spectrum chart, and determine the frequency-domain feature as a target feature.

The frequency-domain feature includes ultra low frequency (very low frequency, ULF) power, low frequency (low frequency, LF) power, high frequency (high frequency, HF) power, or a ratio (LF/HF) of the low frequency power to the high frequency power. The VLF is ultra low frequency power, which is power in a frequency band of 0.003 to 0.04 Hertz. The LF is low frequency power, which is power in a frequency band of 0.04 to 0.15 Hertz. The HF is high frequency power, which is power in a frequency band of 0.15 to 0.4 Hertz. The LF/HF is a ratio of the low frequency power to the high frequency power.

In an example, the frequency-domain feature may include the ULF power, the HF power, and the LF power.

In another example, the frequency-domain feature may include the ULF power and the LF/HF.

In still another example, the frequency-domain feature may include the ULF power, the HF power, the LF power, and the LF/HF.

According to the heart rhythm abnormality identification method provided in this application, the target feature includes the frequency-domain feature and the distribution feature of the heart rate data in the Poincare plot, so that features are extracted in a plurality of dimensions, which further helps represent a distribution feature of the heart rate data, and helps improve accuracy of heart rhythm abnormality identification.

With reference to the first aspect, in some implementations of the first aspect, the heart rate data is obtained based on the IBI data of the PPG signal; and the method further includes: determining a sample entropy and/or a Shannon entropy based on the IBI data; and determining the sample entropy and/or the Shannon entropy as the target feature.

The wearable device may extract a non-linear feature in a non-linear dimension based on the IBI data, and determine the non-linear feature as the target feature. The non-linear feature may include a sample entropy and/or a Shannon entropy.

According to the heart rhythm abnormality identification method provided in this application, the target feature includes the non-linear feature and the distribution feature of the heart rate data in the Poincare plot, so that features are extracted in a plurality of dimensions, which further helps represent a distribution feature of the heart rate data, and helps improve accuracy of heart rhythm abnormality identification.

With reference to the first aspect, in some implementations of the first aspect, the obtaining heart rate data of a user includes: collecting a PPG signal of the user; determining whether the user is in a static state when the PPG signal is collected; and determining the heart rate data based on IBI data in the PPG signal if the user is in the static state when the PPG signal is collected.

The wearable device may collect acceleration data by using an acceleration sensor when collecting a PPG signal of the user, and determine, based on the acceleration data, whether the wearable device of the user is in the static state, to further determine whether the user is in the static state. If the user is in the static state when the PPG signal is collected, it may indicate that the PPG signal at this time is accurate and can be used, and heart rate data can be determined based on IBI data in the PPG signal.

According to the heart rhythm abnormality identification method provided in this application, whether the user is in the static state is determined during PPG signal collection, which helps ensure that all subsequently used PPG signals are collected when the user is in the static state, helps ensure quality of the PPG signals, and helps improve accuracy of subsequent identification as a feature extracted based on a PPG signal of good quality can facilitate accurate identification.

With reference to the first aspect, in some implementations of the first aspect, the method further includes: if the user is not in the static state when the PPG signal is collected, determining whether a duration within which the user is not in the static state exceeds a first duration; and determining the heart rate data based on the IBI data in the PPG signal if the duration within which the user is not in the static state does not exceed the first duration.

The duration within which the user is not in the static state may be referred to as a non-static duration. The first duration in embodiments is a preset duration A.

If the user is not in the static state when the PPG signal is collected, whether the non-static duration exceeds the preset duration A is determined. If the non-static duration exceeds the preset duration A, it may indicate that the wearable device keeps in a non-static state within the preset duration A, and is not suitable for heart rhythm detection. If the non-static duration does not exceed the preset duration A, the wearable device may determine heart rate data based on IBI data in the PPG signal.

According to the heart rhythm abnormality identification method provided in this application, during PPG signal collection, if the duration within which the user is not in the static state does not exceed the first duration, a PPG signal collected within this duration can be used, and if the duration within which the user is not in the static state exceeds the first duration, the PPG signal collected within this duration cannot be used, instead of discarding all PPG signals in the non-static state, so that fault tolerance for PPG signal collection can be provided by using the first duration, which helps quickly complete PPG signal collection for a subsequent process, and helps improve efficiency of heart rhythm abnormality identification.

With reference to the first aspect, in some implementations of the first aspect, the obtaining heart rate data of a user includes: collecting a PPG signal of the user; and determining the heart rate data based on IBI data in the PPG signal if a peak value of the PPG signal is greater than or equal to a preset peak value.

The PPG signal is periodic, and the wearable device may determine whether peak value data in each periodicity of the PPG signal exceeds the preset peak value. If the peak value data in each periodicity exceeds the preset peak value, it may indicate that signal quality in each periodicity is good. If peak value data in one or more periodicities in the peak value data does not exceed the preset peak value, it may indicate that signal quality is unstable and the PPG signal may be discarded.

According to the heart rhythm abnormality identification method provided in this application, when a peak value of a PPG signal is greater than or equal to the preset peak value, heart rate data is determined based on IBI data in the PPG signal, which helps ensure quality of the PPG signal, and helps improve accuracy of subsequent identification as a feature extracted based on a PPG signal of good quality can facilitate accurate identification.

With reference to the first aspect, in some implementations of the first aspect, the collecting a PPG signal of the user includes: collecting a PPG signal of the user if the user keeps in the static state within a second duration.

The second duration may be a preset duration C in embodiments. The wearable device obtains acceleration data within the preset duration C by using an acceleration sensor, and determines, based on the acceleration data within the preset duration C, whether the wearable device keeps in the static state within the preset duration C. If the wearable device keeps in the static state within the preset duration C, the wearable device may collect a PPG signal of the user. If the wearable device does not keep in the static state within the preset duration C, or the wearable device is not in the static state at one or more moments within the preset duration C, the wearable device may delete a collected PPG signal.

According to the heart rhythm abnormality identification method provided in this application, before PPG signal collection, whether the user keeps in the static state within the second duration may be determined first. If the user keeps in the static state within the second duration, it may indicate that a heart rhythm abnormality identification condition is met, and heart rhythm abnormality identification may be performed. If the user does not keep in the static state within the second duration, it may indicate that the heart rhythm abnormality identification condition is not met, and heart rhythm abnormality identification may not be performed. In this manner, heart rhythm abnormality identification is performed when the heart rhythm abnormality identification condition is met, which helps ensure accuracy of heart rhythm abnormality identification.

With reference to the first aspect, in some implementations of the first aspect, the identification model is a random forest, a gradient boosting decision tree, extreme gradient boosting, or a support vector machine.

With reference to the first aspect, in some implementations of the first aspect, the identification result is a sinus rhythm, premature beat, or atrial fibrillation.

According to a second aspect, this application provides a wearable device. The wearable device includes: an obtaining module and a processing module. The obtaining module is configured to obtain heart rate data of a user. The processing module is configured to: determine a target feature of the heart rate data based on the heart rate data and a Poincare plot, where the target feature includes a first feature and a second feature, the first feature is a standard deviation of a distance from an origin of the Poincare plot to a first straight line, the first straight line is a straight line formed by first heart rate data located in an area 2 in the Poincare plot and second heart rate data located in an area 4 in the Poincare plot, an obtaining time of the first heart rate data is earlier than an obtaining time of the second heart rate data, the obtaining time of the first heart rate data is adjacent to the obtaining time of the second heart rate data, the second feature is a standard deviation of a distance from the origin of the Poincare plot to a second straight line, the second straight line is a straight line formed by third heart rate data located in the area 4 in the Poincare plot and fourth heart rate data located in the area 2 in the Poincare plot, an obtaining time of the third heart rate data is earlier than an obtaining time of the fourth heart rate data, and the obtaining time of the third heart rate data is adjacent to the obtaining time of the fourth heart rate data; and input the target feature to an identification model, to obtain an identification result, where the identification model is configured to identify, based on the input, whether a heart rhythm is abnormal.

With reference to the second aspect, in some implementations of the second aspect, the target feature further includes a third feature and a fourth feature, the third feature is a modulus of an angle corresponding to the first straight line, and the fourth feature is a modulus of an angle corresponding to the second straight line.

With reference to the second aspect, in some implementations of the second aspect, the target feature further includes a fifth feature and a sixth feature, the fifth feature is a standard deviation of the angle corresponding to the first straight line, and the sixth feature is a standard deviation of the angle corresponding to the second straight line.

With reference to the second aspect, in some implementations of the second aspect, the target feature further includes a seventh feature, an eighth feature, and a ninth feature, the seventh feature is a ratio of a quantity of data pieces of the heart rate data that are located in an area 0 in the Poincare plot to a total quantity of pieces of the heart rate data, the eighth feature is a standard deviation of an angle that uses heart rate data located in the area 2 as a vertex in three pieces of adjacent heart rate data of the heart rate data that are respectively located in an area 1, the area 2, and an area 3 in the Poincare plot, and the ninth feature is a standard deviation of an angle that uses heart rate data located in the area 4 as a vertex in three pieces of adjacent heart rate data of the heart rate data that are respectively located in an area 6, the area 4, and an area 5 in the Poincare plot.

With reference to the second aspect, in some implementations of the second aspect, the heart rate data is obtained based on inter-beat interval IBI data of a photoplethysmography PPG signal, and the target feature further includes at least one of the following: a ratio of a quantity of differences greater than 50 milliseconds between adjacent pieces of data in the IBI data to a total quantity of pieces in the IBI data, a mean value of the IBI data, a median of the IBI data, a standard deviation of the IBI data, a root mean square of differences between adjacent pieces of data in the IBI data, or a standard deviation of the differences between the adjacent pieces of data in the IBI data.

With reference to the second aspect, in some implementations of the second aspect, the heart rate data is obtained based on the IBI data of the PPG signal. The processing module is further configured to: generate a power spectrum chart based on the IBI data; and determine at least one of a ratio of low frequency power to high frequency power, ultra low frequency power, the low frequency power, or the high frequency power in the power spectrum chart as the target feature.

With reference to the second aspect, in some implementations of the second aspect, the heart rate data is obtained based on the IBI data of the PPG signal. The processing module is further configured to: determine a sample entropy and/or a Shannon entropy based on the IBI data; and determine the sample entropy and/or the Shannon entropy as the target feature.

With reference to the second aspect, in some implementations of the second aspect, the obtaining module is further configured to collect a PPG signal of the user. The processing module is further configured to: determine whether the user is in a static state when the PPG signal is collected; and determine the heart rate data based on IBI data in the PPG signal if the user is in a static state when the PPG signal is collected.

With reference to the second aspect, in some implementations of the second aspect, the processing module is further configured to: if the user is not in the static state when the PPG signal is collected, determine whether a duration within which the user is not in the static state exceeds a first duration; and determine the heart rate data based on the IBI data in the PPG signal if the duration within which the user is not in the static state does not exceed the first duration.

With reference to the second aspect, in some implementations of the second aspect, the obtaining module is further configured to collect a PPG signal of the user. The processing module is further configured to determine the heart rate data based on IBI data in the PPG signal if a peak value of the PPG signal is greater than or equal to a preset peak value.

With reference to the second aspect, in some implementations of the second aspect, the obtaining module is further configured to collect a PPG signal of the user if the user keeps in the static state within a second duration.

With reference to the second aspect, in some implementations of the second aspect, the identification model is a random forest, a gradient boosting decision tree, extreme gradient boosting, or a support vector machine.

With reference to the second aspect, in some implementations of the second aspect, the identification result is a sinus rhythm, premature beat, or atrial fibrillation.

According to a third aspect, this application provides a wearable device. The wearable device includes: a processor and a memory. The memory stores computer-executable instructions. The processor executes the computer-executable instructions stored in the memory, to enable the wearable device to perform the method according to the first aspect.

According to a fourth aspect, this application provides a computer-readable storage medium. The computer-readable storage medium stores a computer program. When the computer program is executed by a processor, the method according to the first aspect is implemented.

According to a fifth aspect, this application provides a computer program product. The computer program product includes a computer program, and when the computer program is run, a computer is enabled to perform the method according to the first aspect.

According to a sixth aspect, this application provides a chip. The chip includes a processor, and the processor is configured to invoke a computer program in a memory to perform the method according to the first aspect.

It should be understood that, technical solutions of the third aspect to the sixth aspect of this application correspond to those of the first aspect of this application, and beneficial effects achieved by the aspects and corresponding feasible implementations are similar, which are not detailed again.

## BRIEF DESCRIPTION OF DRAWINGS

FIG. 1 is a diagram of a hardware structure of a wearable device according to an embodiment of this application;

FIG. 2 is a schematic flowchart of a heart rhythm abnormality identification method according to an embodiment of this application;

FIG. 3 is a diagram of peak value data extraction according to an embodiment of this application;

FIG. 4 is a diagram of feature extraction according to an embodiment of this application;

FIG. 5 is another diagram of feature extraction according to an embodiment of this application;

FIG. 6 is a block diagram of heart rhythm abnormality identification according to an embodiment of this application;

FIG. 7 is a schematic flowchart of a heart rhythm abnormality identification method according to an embodiment of this application;

FIG. 8 is a block diagram of machine model training according to an embodiment of this application;

FIG. 9 is a diagram of PPG signals of different heart rhythms according to an embodiment of this application;

FIG. 10 is a diagram of area distribution of different PPG signals in a Poincare plot according to an embodiment of this application;

FIG. 11 is another diagram of area distribution of different PPG signals in a Poincare plot according to an embodiment of this application;

FIG. 12 is a diagram of a wearable device according to an embodiment of this application; and

FIG. 13 is a diagram of another wearable device according to an embodiment of this application.

## DESCRIPTION OF EMBODIMENTS

To clearly describe technical solutions in embodiments of this application, words such as "first" and "second" are used in embodiments of this application to distinguish between same items or similar items that provide basically same functions or effects. A person skilled in the art may understand that the words such as "first" and "second" do not limit a quantity or an execution sequence, nor indicate a definite difference.

In embodiments of this application, words such as "example" or "for example" are used to represent giving an example, an illustration, or a description. Any embodiment or design scheme described as an "example" or "for example" in this application should not be explained as being more preferred or advantageous than another embodiment or design scheme. To be precise, use of the word such as "example" or "for example" is intended to present a relative concept in a specific manner.

In embodiments of this application, "at least one" means one or more, and "a plurality of" means two or more. The term "and/or" describes an association relationship between associated objects and represents that three relationships may exist. For example, A and/or B may represent the following cases: only A exists, both A and B exist, and only B exists, where A and B may be singular or plural. The character "/" in this specification generally indicates an "or" relationship between the associated objects. The representation "at least one of the following items (pieces)" or a similar representation thereof indicates any combination of these items, including a single item (piece) or any combination of a plurality of items (pieces). For example, at least one of a, b, or c may represent: a, b, c, a-b, a-c, b-c, or a-b-c, where a, b, and c may be singular or plural.

It should be noted that, "when ..." in embodiments of this application may be an instantaneous time upon occurrence of a case, or may be a period of time after occurrence of the case. This is not specifically limited in embodiments of this application. In addition, a display interface provided in embodiments of this application is only used as an example, and the display interface may alternatively include more or less content.

Professional terms and concepts in embodiments of this application are first described.

1. Photoplethysmography (photoplethysmography, PPG)

The PPG is a method of measuring, based on a light-emitting diode (light-emitting diode, LED) light source and a detector, light attenuated after reflection and absorption of a blood vessel and tissue of a human body, recording a beat status of the blood vessel, and measuring a pulse wave.

2. Inter-beat interval (inter-beat interval, IBI)

The inter-beat interval is a duration between peak values of two adjacent pulses in a PPG signal, and may be used to calculate a heart rate.

3. Heart rate

The heart rate is a quantity of beats per minute of a normal person in a resting state, also referred to as a resting heart rate, and is usually 60 to 100 beats/minute. Heart rate changes are closely related to heart diseases. Most heart rates exceeding 160 beats/minute or less than 40 beats/minute are seen in patients of heart diseases, who are often accompanied by discomfort such as palpitation or chest distress, and should undergo detailed inspection early to get treatment for causes.

4. Heart rhythm

The heart rhythm is a tempo of heartbeat. A normal heart rhythm originates from a sinoatrial node at a frequency of 60 to 100 beats/minute, and is regular. Impulse of the sinoatrial node sequentially excites atria and ventricles via a normal atrioventricular conduction system with a constant conduction time (approximately 0.12 to 1.21 seconds). A conduction time of the impulse reaching ventricular myocardium via bundle branches and their tributaries and Purkinje fibers is also constant (<0.10 second). However, when any one of the heart rhythm origin, the heartbeat frequency and rhythm, and the impulse conduction is abnormal, an abnormal heart rhythm occurs. In short, if the tempo of the heartbeat is regular, the heart rhythm is normal, and if the tempo of the heartbeat is irregular, the heart rhythm is abnormal.

5. Sinus rhythm

The sinus rhythm is manifestation of a normal heart rate of a normal person. A normal heart surely has a normal sinoatrial node, and the normal sinoatrial node has strong autorhythmicity. Any heart rhythm formed by excitation of the sinoatrial node is collectively referred to as the sinus rhythm.

6. Premature beat

The premature beat (premature beat), also referred to as a pre-systole or an extra-systole, and briefly referred to as premature beat, is a premature ectopic heartbeat, which may be classified into the following four types based on origins: a sinus type, an atrial type, an atrioventricular junctional type, and a ventral type. The ventricular type is the most common, followed by the atrial type. Sinus premature beat is rare. The premature beat is a common ectopic heart rhythm, and may occur based on a sinus rhythm or an ectopic (for example, atrial fibrillation) heart rhythm. The premature beat may occur occasionally or frequently, and may occur irregularly or regularly after each or several normal beats, forming bigeminy, trigeminy, or coupled premature beat.

7. Atrial fibrillation

The atrial fibrillation, which may be briefly referred to as atrial fibrillation, is the most common persistent abnormal heart rhythm. The atrial fibrillation may be classified into paroxysmal atrial fibrillation, persistent atrial fibrillation, and permanent atrial fibrillation based on durations. Usually, it is considered that the paroxysmal atrial fibrillation usually lasts less than 48 hours, from which a person can recover to the sinus rhythm within 7 days; the persistent atrial fibrillation lasts for more than 7 days, from which a person requires medication or electric shock to recover to the sinus rhythm; and the permanent atrial fibrillation means that a person cannot recover to the sinus rhythm or relapses within 24 hours after recovery.

With the development of wearable devices, a wearable device can support more functions. Currently, the wearable device can detect a heart rhythm of a user, to determine whether the heart rhythm of the user is a normal heart rhythm or an abnormal heart rhythm. The abnormal heart rhythm may include types such as premature beat, atrial fibrillation, atrial flutter, atrioventricular conduction block, and bundle branch conduction block.

A common heart rhythm abnormality identification method is that the wearable device calculates an average heart rate based on a photoplethysmography (photoplethysmography, PPG) signal, and identifies, based on the average heart rate, whether the heart rhythm of the user is a normal heart rhythm or an abnormal heart rhythm. However, a change in the average heart rate is not a reliable flag of an abnormal heart rhythm, resulting in much misidentification of the wearable device and low identification accuracy. For example, the wearable device mistakenly identifies a normal heart rhythm as an abnormal heart rhythm (such as premature beat), or the wearable device mistakenly identifies an abnormal heart rhythm (such as atrial fibrillation) as a normal heart rhythm.

In view of this, embodiments of this application provide a heart rhythm abnormality identification method and a wearable device. The wearable device may collect a PPG signal within a period of time when the wearable device is static, extract a plurality of IBIs based on the PPG signal within the period of time, then calculate a plurality of heart rates based on each of the plurality of IBIs, calculate differences between adjacent heart rates of the plurality of heart rates, then construct two-dimensional coordinate points based on adjacent differences, extract a statistical feature based on area distribution of the two-dimensional coordinate points in a Poincare plot (poincare plot), and finally input the statistical features to a machine learning model, to obtain an identification result. The statistical feature is determined based on a distribution feature, in the Poincare plot, of adjacent differences between heart rates calculated by using a PPG signal corresponding to an abnormal heart rhythm. In this implementation, compared with using an average heart rate to represent an abnormal heart rhythm, using a statistical feature to represent an abnormal heart rhythm helps improve accuracy of heart rhythm abnormality identification.

The heart rhythm abnormality identification method provided in embodiments of this application may be performed by a wearable device, for example, a smart watch, a smart band, or an augmented reality (augmented reality, AR) wearable device.

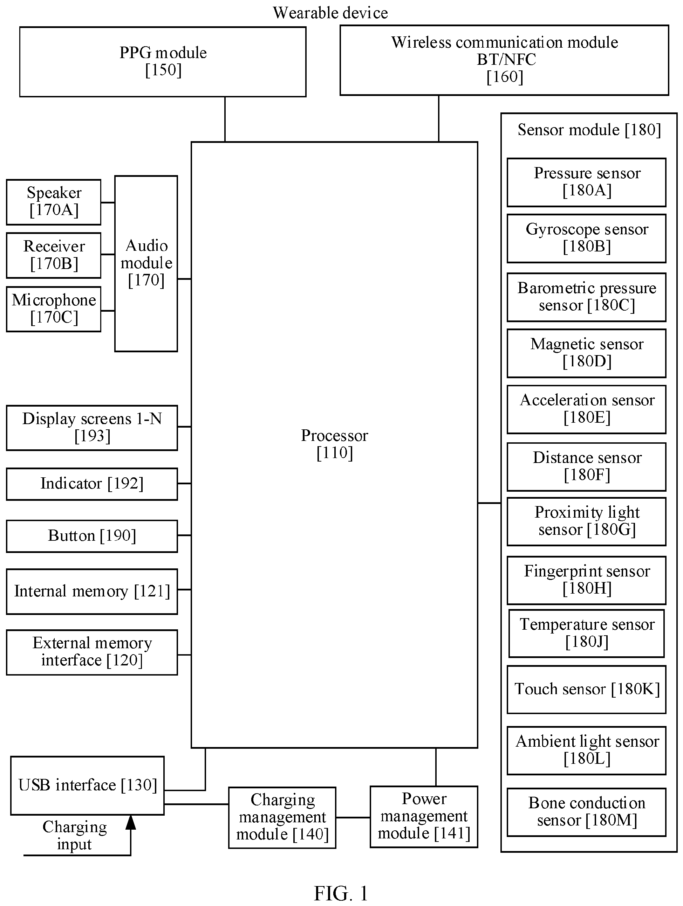

To better understand embodiments of this application, the following describes a hardware structure of the wearable device in embodiments of this application. For example, FIG. 1 is a diagram of a hardware structure of a wearable device according to an embodiment of this application.

The wearable device may include a processor 110, an external memory interface 120, an internal memory 121, a universal serial bus (universal serial bus, USB) interface 130, a charging management module 140, a power management module 141, a PPG module 150, a wireless communication module 160, an audio module 170, a speaker 170A, a receiver 170B, a microphone 170C, a sensor module 180, a button 190, an indicator 192, a display screen 193, and the like.

It may be understood that the schematic structure in this embodiment of this application does not constitute a specific limitation on the wearable device. In some other embodiments of this application, the wearable device may include more or fewer components than those shown in the figure, or have some components combined, or have some components split, or have a different component arrangement. The components shown in the figure may be implemented by hardware, software, or a combination of software and hardware.

The processor 110 may include one or more processing units. Different processing units may be independent components, or may be integrated into one or more processors. A memory may be further disposed in the processor 110, to store instructions and data. In this embodiment of this application, the processor 110 may be configured to perform calculation and extract IBIs based on a PPG signal, calculate heart rates and differences between adjacent heart rates based on the IBIs, extract a statistical feature based on adjacent differences, and the like. The processor 110 may be further configured to input the statistical feature to a machine learning model, to obtain an identification result.

The external memory interface 120 may be configured to connect to an external storage card, such as a Micro SD card, to expand a storage capability of the wearable device. The external memory card communicates with the processor 110 through the external memory interface 120, to implement a data storage function. For example, music, video, or other files are stored in the external memory card.

The internal memory 121 may be configured to store computer-executable program code. The executable program code includes instructions. The internal memory 121 may include a program storage area and a data storage area.

The USB interface 130 is an interface that complies with a USB standard specification, and may be specifically a Mini USB interface, a Micro USB interface, a USB Type C interface, or the like. The USB interface 130 may be configured to connect to a charger to charge the wearable device.

The charging management module 140 is configured to receive a charging input from a charger. The charger may be a wireless charger, or may be a wired charger. The power management module 141 is configured to connect to the charging management module 140 and the processor 110.

The PPG module 150 includes an LED module and a photo diode (photo diode, PD) module. The LED module may include one or more LEDs. This is not limited in this embodiment of this application. The LED may be a tri-color LED, and the LED may emit light sources such as red light, green light, and infrared light. The PD module may include one or more PDs. This is not limited in this embodiment of this application. The PD may be configured to receive an optical signal and process the optical signal into an electrical signal. For example, in a scenario of collecting a PPG signal, the PD may receive an optical signal reflected by skin tissue, and process the signal into an electrical signal.

The wireless communications module 160 may provide a wireless communication solution applied to the wearable device, such as Bluetooth (bluetooth, BT) and near field communication (near field communication, NFC).

The audio module 170 is configured to convert digital audio information into an analog audio signal for output, and convert an analog audio input into a digital audio signal. The speaker 170A, also referred to as a "loudspeaker", is configured to convert an electrical audio signal into a sound signal. Music may be listened to or a hands-free call may be answered by using the speaker 170A in the wearable device. The receiver 170B, also referred to as a "phone receiver", is configured to convert an audio electrical signal into a sound signal. When the wearable device is used to answer a call or receive voice information, the receiver 170B may be placed close to an ear to listen to a voice. The microphone 170C, also referred to as a "mic" or a "mike", is configured to convert a sound signal into an electrical signal.

The wearable device may use the audio module 170, the speaker 170A, the receiver 170B, the microphone 170C, an application processor, and the like, to implement an audio function, for example, music playing or sound recording.

The sensor module 180 may include a pressure sensor 180A, a gyroscope sensor 180B, a barometric pressure sensor 180C, a magnetic sensor 180D, an acceleration sensor (accelerometer, Acc) 180E, a distance sensor 180F, a proximity light sensor 180G, a fingerprint sensor 180H, a temperature sensor 180J, a touch sensor 180K, an ambient light sensor 180L, a bone conduction sensor 180M, and the like.

The pressure sensor 180A is configured to sense a pressure signal, and can convert the pressure signal into an electrical signal. In some embodiments, the pressure sensor 180A may be disposed on the display screen 193. The gyroscope sensor 180B may be configured to determine a motion attitude of the wearable device. The barometric pressure sensor 180C is configured to measure barometric pressure. The magnetic sensor 180D includes a Hall sensor. The acceleration sensor 180E may detect magnitudes of accelerations of the wearable device in various directions (generally on three axes). The distance sensor 180F is configured to measure a distance. The proximity light sensor 180G may include, for example, an LED and an optical detector, for example, a photo diode. The ambient light sensor 180L is configured to sense intensity of ambient light. The fingerprint sensor 180H is configured to collect a fingerprint. The temperature sensor 180J is configured to detect a temperature. The touch sensor 180K is also referred to as a "touch component". The touch sensor 180K may be disposed on the display screen 193. The touch sensor 180K and the display screen 193 constitute a touchscreen, which is also referred to as a "touch control screen". The bone conduction sensor 180M may obtain a vibration signal.

The button 190 includes a power button, a selection button, a volume button, and the like. The button 190 may be a mechanical button, or may be a touch button. The wearable device may receive a key input, and generate a key signal input related to user setting and function control of the wearable device. The indicator 192 may be an indicator light, and may be configured to indicate a charging status or a power change, and indicate a message, a missed call, a notification, and the like.

The display screen 193 is configured to display an image, a video, and the like. The display screen 193 includes a display panel. In some embodiments, the wearable device may include one or N display screens 193. N is a positive integer greater than 1.

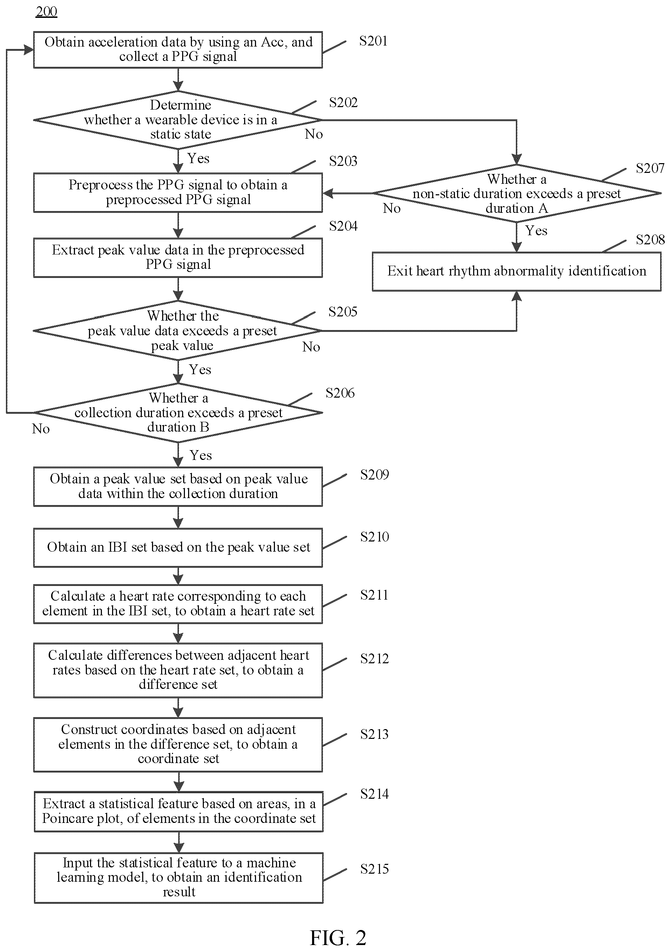

FIG. 2 is a schematic flowchart of a heart rhythm abnormality identification method 200 according to an embodiment of this application. The method 200 may be performed by a wearable device, for example, a smart watch. A diagram of a hardware structure of the wearable device may be shown in FIG. 1, but this embodiment of this application is not limited thereto.

As shown in FIG. 2, the method 200 may include the following steps.

S201. Obtain acceleration data by using an acceleration sensor (Acc), and collect a PPG signal.

There may be a plurality of trigger conditions of S201. In a possible example, a trigger condition of S201 is that the wearable device detects an operation of a user triggering a heart rhythm detection option. To be specific, the wearable device detects the operation of the user triggering the heart rhythm detection option, and performs S201 in response to the operation of the user triggering the heart rhythm detection option. In another possible example, a trigger condition of S201 is that the wearable device detects that a heart rhythm detection time arrives. The wearable device may provide a function of periodic heart rhythm detection or regular heart rhythm detection for the user. When the wearable device detects that the function of periodic heart rhythm detection or regular heart rhythm detection is enabled, the wearable device may monitor, in real time, whether the heart rhythm detection time arrives. When the wearable device detects that the heart rhythm detection time arrives, S201 is performed.

The acceleration sensor (Acc) may be the acceleration sensor 180E in FIG. 1, but this embodiment of this application is not limited thereto. The wearable device may obtain acceleration data of the wearable device on three axes (generally an X axis, a Y axis, and a Z axis) by using the Acc. If the wearable device obtains, by using the Acc, acceleration data $x_0$ of the wearable device on the x axis, acceleration data $y_0$ on the Y axis, and acceleration data $z_0$ on the z axis, the acceleration data obtained by the wearable device by using the Acc is $(x_0, y_0, z_0)$.

The wearable device may obtain acceleration data at a moment or acceleration data within a period of time by using the Acc. This is not limited in this embodiment of this application.

If the wearable device obtains acceleration data on the three axes at a moment, the wearable device may determine acceleration data of the wearable device at this moment based on the acceleration data on the three axes and a body diagonal formula $l=\sqrt{a^2+b^2+c^2}$ .

For example, the wearable device detects the operation of the user triggering the heart rhythm detection option, and in response to the operation of the user triggering the heart rhythm detection option, the wearable device obtains a piece of acceleration data $(x_0, y_0, z_0)$ on the three axes by using the Acc. The wearable device may obtain acceleration data $\sqrt{x_0^2+y_0^2+z_0^2}$ at this moment.

If the wearable device obtains acceleration data within a period of time, the wearable device may determine a maximum value of all acceleration data on the three axes within this period of time as acceleration data of the wearable device within this period of time. This period of time may be 1 second, 0.5 second, or the like. This is not limited in this embodiment of this application.

For example, the wearable device obtains 200 pieces of acceleration data on the three axes within 1 second: $(x_1, y_1, z_1)$, $(x_2, y_2, z_2)$, ..., $(x_{200}, y_{200}, z_{200})$, and the wearable device may determine a maximum value of $(x_1, y_1, z_1)$, $(x_2, y_2, z_2)$, ..., $(x_{200}, y_{200}, z_{200})$ as acceleration data of the wearable device within this period of time, that is,
$$\max(\sqrt{x_1^2+y_1^2+z_1^2},\sqrt{x_2^2+y_2^2+z_2^2},...,\sqrt{x_{200}^2+y_{200}^2+z_{200}^2})$$ .

While collecting the acceleration data, the wearable device may further collect a PPG signal. For example, the wearable device may collect a PPG signal by using the PPG module 150 shown in FIG. 1. A duration of collecting the acceleration data by the wearable device is the same as a duration of collecting the PPG signal.

S202. Determine, based on the acceleration data, whether the wearable device is in a static state.

The wearable device may compare the acceleration data with an acceleration threshold. If the acceleration data is less than or equal to the acceleration threshold, it is determined that the wearable device is in the static state. If the acceleration data is greater than the acceleration threshold, it is determined that the wearable device is in a non-static state.

For example, the wearable device may normalize the acceleration data $\sqrt{x_0^2+y_0^2+z_0^2}$ or $\max(\sqrt{x_1^2+y_1^2+z_1^2},\sqrt{x_2^2+y_2^2+z_2^2},...,\sqrt{x_{200}^2+y_{200}^2+z_{200}^2})$ in the foregoing example into a value between 0 and 10, and the acceleration threshold may be 5. If the acceleration data is less than or 5, it is determined that the wearable device is in the static state. If the acceleration data is greater than 5, it is determined that the wearable device is in the non-static state.

If the wearable device is in the static state, it may indicate that the PPG signal is collected when the wearable device is in the static state and may be used for subsequent calculation. For example, the wearable device may preprocess the PPG signal, that is, perform S203. If the wearable device is in the non-static state, it may indicate that the PPG signal is collected when the wearable device is in the non-static state. The wearable device may determine whether a duration within which the wearable device is in the non-static state exceeds a preset duration A (for example, $L_2$ seconds), that is, perform S207.

According to the method provided in this embodiment of this application, during PPG signal collection, whether the wearable device is in the static state is determined by using the acceleration data, which helps ensure that all subsequently used PPG signals are collected when the wearable device is in the static state, and helps ensure quality of the PPG signals, and helps improve accuracy of subsequent identification as a feature extracted based on a PPG signal of good quality can facilitate accurate identification.

S203. If the wearable device is in the static state, preprocess the PPG signal to obtain a preprocessed PPG signal.

The preprocessing may be performing denoising through filtering. For example, if the wearable device is in the static state, the wearable device may denoise the PPG signal through low-pass filtering and high-pass filtering, to obtain a denoised PPG signal.

The PPG signal is periodic. Therefore, the PPG signal may be information within a plurality of periodicities. The preprocessed PPG signal and the PPG signal are the same in periodicities.

According to the method provided in this embodiment of this application, before a features is extracted from the PPG signal, the PPG signal is first preprocessed to remove some impurity data, which helps avoid impact of the impurity data on the extracted feature, and helps improve accuracy of subsequent identification.

It should be noted that, the preprocessing performed by the wearable device on the PPG signal in the method 200 is optional.

S204. Extract peak value data in the preprocessed PPG signal.

The preprocessed PPG signal may include a plurality of periodicities, each of the plurality of periodicities has a peak value, and the wearable device may extract peak value data in each periodicity from the preprocessed PPG signal. If the preprocessed PPG signal includes n periodicities, the peak value data may include n peak values. The peak value data may also be referred to as amplitude data. This is not limited in this embodiment of this application.

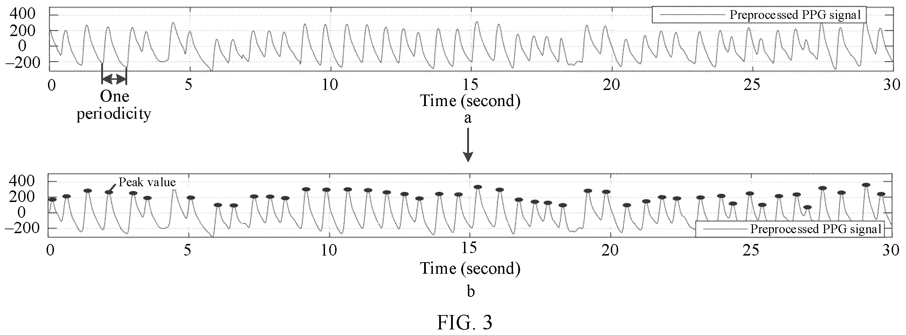

For example, FIG. 3 is a diagram of peak value data extraction. As shown in a of FIG. 3, the preprocessed PPG signal may be displayed on a filter as a waveform shown in a of FIG. 3, a horizontal axis is time, whose unit may be second, and a vertical axis is amplitude. The preprocessed PPG signal may include a plurality of periodicities, and each periodicity has a peak value. The wearable device may extract peak value data in each periodicity from the preprocessed PPG signal shown in a of FIG. 3, and may display b of FIG. 3. As shown in b of FIG. 3, the peak value in each periodicity is represented by a black dot.

S205. Determine whether the peak value data all exceeds a preset peak value.

The wearable device may determine whether the peak value data in each periodicity exceeds the preset peak value. If the peak value data in each periodicity exceeds the preset peak value, it may indicate that signal quality in each periodicity is good. If peak value data in one or more periodicities in the peak value data does not exceed the preset peak value, it may indicate that signal quality is unstable and the PPG signal may be discarded.

If the peak value data in each periodicity exceeds the preset peak value, the wearable device may determine whether data collection is still required, for example, determine whether a duration of continuously collecting a PPG signal (which may be referred to as a collection duration for short) exceeds a preset duration B, that is, perform S206. If peak value data in one or more periodicities in the peak value data does not exceed the preset peak value, the wearable device may exit heart rhythm abnormality identification, that is, perform S208. If peak value data in one or more periodicities in the peak value data does not exceed the preset peak value, the wearable device may further output prompt information to prompt the user that signal quality is not good and recommend the user to wear the wearable device correctly.

S206. If the peak value data all exceeds the preset peak value, determine whether a collection duration exceeds a preset duration B.

If the collection duration (for example, $L_3$ seconds) does not exceed the preset duration B (for example, $L_1$ seconds), the wearable device may continue to collect a PPG signal, and determine, based on acceleration data obtained when the PPG signal is collected, whether the wearable device is in the static state, that is, perform S201 and S202. If the collection duration exceeds the preset duration B, the wearable device may perform feature extraction based on peak value data collected within the preset duration B, that is, perform S209.

It may be understood that, if the wearable device collects a PPG signal at a moment in S201, the collection duration is a sum of a plurality of moments. If the wearable device collects a PPG signal within a period of time in S201, the collection duration is a sum of a plurality of periods of time.

S207. If the wearable device is in the non-static state, determine whether a non-static duration exceeds a preset duration A.

The non-static duration is a duration within which the wearable device continuously determines that the wearable device is in the non-static state. For example, if the wearable device keeps determining, within 3 seconds based on obtained acceleration data, that the wearable device is in the non-static state, the non-static duration is 3 seconds.

If the non-static duration exceeds the preset duration A, it may indicate that the wearable device keeps in the non-static state within the preset duration A, and is not suitable for heart rhythm detection, and the wearable device may exit heart rhythm abnormality identification, that is, perform S208. If the non-static duration does not exceed the preset duration A, the wearable device may preprocess the PPG signal, that is, perform S203.

It may be understood that S207 and S203 are parallel solutions. It should be noted that, the preset duration A is less than the preset duration B.

According to the method provided in this embodiment of this application, during PPG signal collection, if the non-static duration does not exceed the preset duration A, a PPG signal collected within the non-static duration can be used, and if the non-static duration exceeds the preset duration A, the PPG signal collected within the non-static duration cannot be used, instead of discarding all PPG signals in the non-static state, so that fault tolerance for PPG signal collection can be provided by using the preset duration A, which helps quickly complete PPG signal collection for a subsequent process, and helps improve efficiency of heart rhythm abnormality identification.

S208. If the non-static duration exceeds the preset duration A, exit heart rhythm abnormality identification.

If the non-static duration exceeds the preset duration A, the wearable device may exit heart rhythm abnormality identification, and may further output prompt information to prompt the user that the wearable device is in the non-static state and recommend the user to keep the wearable device static. The wearable device may further clear the collected PPG signal.

S209. If the collection duration exceeds the preset duration B, obtain a peak value set based on peak value data within the collection duration.

The collection duration is a duration of continuously collecting a PP signal. If the collection duration exceeds the preset duration B, the wearable device may obtain the peak value data within the preset duration B. The peak value data may form a peak value set. The peak value set may be presented by $P_m$, but this embodiment of this application is not limited thereto.

For example, the preset duration B is $L_1$ seconds, the collection duration is $L_3$ seconds, $L_3 > L_1$, and the wearable device may form a peak value set by using peak value data collected within $L_3$ seconds. If the peak value data collected within the $L_3$ seconds includes q pieces of peak value data, $P_m = [P_{m1}, P_{m2}, ..., P_{mq}]$. It may be understood that elements in $P_m$ may be positive, negative, or zero.

S210. Extract IBIs based on the peak value set, to obtain an IBI set.

The IBI is a duration between adjacent peak values in the peak value set. The wearable device may calculate durations between adjacent peaks (for example, $P_{m1}$ and $P_{m2}$, and $P_{mq-1}$ and $P_{mq}$) in the peak value set $P_m$. If the IBI set may be represented by **TP**, $TP = [TP_1, TP_1, ..., TP_{q-1}]$. $TP_1$ is equal to a duration between $P_{m1}$ and $P_{m2}$, $TP_2$ is equal to a duration between $P_{m3}$ and $P_{m4}$, ..., and $TP_{q-1}$ is equal to a duration between $P_{mq-1}$ and $P_{mq}$. A unit of the elements in **TP** may be second or millisecond. This is not limited in this embodiment of this application. It may be understood that, the elements in **TP** are all positive values.

S211. Calculate a heart rate corresponding to each element in the IBI set, to obtain a heart rate set.

If the heart rate set may be represented by **HR**, $HR = [HR_1, HR_2, ..., HR_{q-1}]$. If the unit of the elements in the IBI set is millisecond,

$$HR_i = \frac{60 * 1000}{TP_i}$$

. HR represents any element in the **HR,** and $TP_i$ represents an element in **TP** and corresponding to $HR_i$, that is,

$$HR_1 = \frac{60 * 1000}{TP_1}, \quad HR_2 = \frac{60 * 1000}{TP_2}, ..., \text{ and } \quad HR_{q-1} = \frac{60 * 1000}{TP_{q-1}}$$

. If the unit of the elements in the IBI set is second,

$$HR_i = \frac{60}{TP_i}$$

. $HR_i$ represents any element in the **HR,** and $TP_i$ represents an element in **TP** and corresponding to $HR_i$, that is,

$$HR_1 = \frac{60}{TP_1}, \quad HR_2 = \frac{60}{TP_2}, ..., \text{ and } \quad HR_{q-1} = \frac{60}{TP_{q-1}}$$

.

S212. Calculate differences between adjacent heart rates based on the heart rate set, to obtain a difference set.

The wearable device may calculate differences between adjacent heart rates (for example, $HR_1$ and $HR_2$, and $HR_{p-2}$ and $HR_{q-1}$) in the heart rate set **HR,** to obtain a difference set. If the difference set may be represented by $\Delta$**HR**, $\Delta$**HR**$= [\Delta HR_1, \Delta HR_2, ..., \Delta HR_{q-2}]$. $\Delta HR_1 = HR_2 - HR_1$, $\Delta HR_2 = HR_3 - HR_2$, ..., and $\Delta HR_{q-2} = HR_{q-1} - HR_{q-2}$.

It may be understood that elements in $\Delta$**HR** may be positive, negative, or zero.

S213. Construct coordinates based on adjacent elements in the difference set, to obtain a coordinate set.

The wearable device may construct coordinates based on adjacent elements (for example, $\Delta HR_1$ and $\Delta HR_2$, and $\Delta HR_{q-3}$ and $\Delta HR_{q-2}$) in the difference set $\Delta$ HR, to obtain a coordinate set. If the coordinate set may be represented by **W,** **W**$= [W_1, W_2, ..., W_{q-3}]$. $W_1 = (\Delta HR_2, \Delta HR_1)$, $W_2 = (\Delta HR_3, \Delta HR_2)$, ..., and $W_{q-3} = (\Delta HR_{q-2}, \Delta HR_{q-3})$.

S214. Extract a statistical feature based on areas, in a Poincare plot, of elements in the coordinate set.

The Poincare plot uses an origin (0, 0) as a center, whose upper, lower, left, and right sides are bounded by a maximum value of heart rate differences; or uses an origin as a center, whose upper, lower, left, and right sides are not bounded. A horizontal coordinate of the Poincare plot may be represented by $\Delta HR_{i+1}$, and a vertical coordinate may be represented by $\Delta HR_i$.

The Poincare plot may include nine areas: an area 0, an area 1, an area 2, an area 3, an area 4, an area 5, an area 6, an area 7, and an area 8. A range of the area 0 may be $(-5 \leq HR_{i+1} \leq 5, -5 \leq HR_i \leq 5)$, a range of the area 1 may be $(5 < HR_{i+1}, -5 \leq HR_i \leq 5)$, a range of the area 2 may be $(HR_{i+1} < -5, 5 < HR_i)$, a range of the area 3 may be $(-5 \leq HR_{i+1} \leq 5, HR_i < -5)$, a range of the area 4 may be $(5 < HR_{i+1}, HR_i < -5)$, a range of the area 5 may be $(-5 \leq HR_{i+1} \leq 5, 5 < HR_i)$, a range of the area 6 may be $(HR_{i+1} < -5, -5 \leq HR_i \leq 5)$, and a range of the area 7 may be $(5 < HR_{i+1}, 5 < HR_i)$, and a range of the area 8 may be $(HR_{i+1} < -5, HR_i < -5)$.

The wearable device may determine, based on coordinate values of elements in the coordinate set and the ranges of the nine areas, areas of the elements in the Poincare plot.

For example, if $W_1 = (40, 15)$, $W_2 = (3, -4)$, and $W_3 = (-40, 60)$, $W_1$ is located in the area 7 in the Poincare plot, $W_2$ is located in the area 0 in the Poincare plot, and $W_3$ is located in the area 2 in the Poincare plot.

The wearable device may extract a statistical feature based on the areas of the elements in the Poincare plot.

The wearable device may calculate a ratio of a quantity of elements located in the area 0 to a total quantity of elements in the coordinate set **W,** to obtain a feature 1. The feature 1 may be represented by $r_{zero}$, but this embodiment of this application is not limited thereto. The wearable device may construct a triangle by using three adjacent elements in the coordinate set **W** that are respectively located in the area 1, the area 2, and the area 3, and calculate, based on a cosine theorem, an angle that uses the element located in the area 2 as a vertex. If there are a plurality of such angles in the coordinate set **W,** the wearable device may calculate a standard deviation of these angles to obtain a feature 2. The feature 2 may be represented by $sd_{123}$, but this embodiment of this application is not limited thereto. The wearable device may construct a triangle by using three adjacent elements in the coordinate set **W** that are respectively located in the area 6, the area 4, and the area 5, and calculate, based on the cosine theorem, an angle that uses the element located in the area 4 as a vertex. If there are a plurality of such angles in the coordinate set **W,** the wearable device may calculate a standard deviation of these angles to obtain a feature 3. The feature 3 may be represented by $sd_{645}$, but this embodiment of this application is not limited thereto.

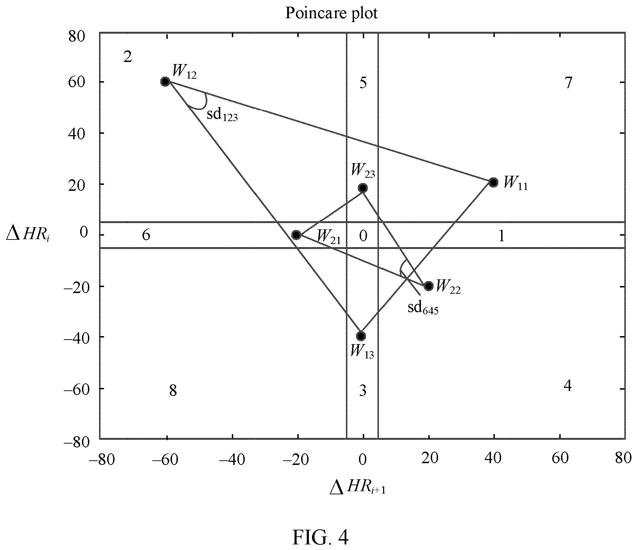

For example, for the coordinate set $\mathbf{W}=[W_1, W_2, ..., W_{q-3}]$, if q is 1003, the coordinate set **W** includes 1003-3=1000 elements. If the quantity of elements in the coordinate set **W** that are located in the area 0 is 300, the feature 1 is $r_{zero}$ 300/1000=0.3=30%. If in the coordinate set **W,** $W_{11}=(40, 20)$ is located in the area 1 in the Poincare plot, $W_{12}=(-60, 60)$ is located in the area 2 in the Poincare plot, $W_{13}=(0, -40)$ is located in the area 3 in the Poincare plot, $W_{21}=(-20, 0)$ is located in the area 6 in the Poincare plot, $W_{22}=(20, -20)$ is located in the area 4 in the Poincare plot, and $W_{23}=(0, 20)$ is located in the area 5 in the Poincare plot, the wearable device may extract the feature 2 based on $W_{11}$, $W_{12}$, and $W_{13}$, and extract the feature 3 based on $W_{21}$, $W_{22}$, and $W_{23}$. A specific extraction manner may be shown in FIG. 4.

The wearable device may construct a triangle $\Delta W_{11}W_{12}W_{13}$ by using $W_{11}$, $W_{12}$, and $W_{13}$ as vertexes. In this case, side lengths of the triangle $\Delta W_{11}W_{12}W_{13}$ are respectively a side length

$$W_{11}W_{12}=\text{a}=\sqrt{(-60-40)^2+(60-20)^2}=\sqrt{11600}$$ , a side length

$$W_{11}W_{13}=\text{b}=\sqrt{(0-40)^2+(-40-20)^2}=\sqrt{5200}$$ , and a side length

$$W_{12}W_{13}=\text{c}=\sqrt{(0-(-60))^2+(-40-60)^2}=\sqrt{13600}$$ .

The wearable device may obtain, based on the cosine theorem, an angle that uses the element $W_{12}$ located in the area 2 as a vertex:

$$\theta_{123}=\arccos\frac{\text{a}^2+\text{c}^2-\text{b}^2}{2*\text{a}*\text{c}}=\arccos\frac{(W_{11}W_{12})^2+(W_{12}W_{13})^2-(W_{11}W_{13})^2}{2*W_{11}W_{12}*W_{12}W_{13}}$$
$$=\arccos\frac{(\sqrt{11600})^2+(\sqrt{13600})^2-(\sqrt{5200})^2}{2*\sqrt{11600}*\sqrt{13600}}$$ .

If there are a plurality of such angles in the coordinate set **W,** the wearable device may calculate a standard deviation of these angles to obtain the feature 2.

The wearable device may construct a triangle $\Delta W_{21}W_{22}W_{23}$ by using $W_{21}$, $W_{22}$, and $W_{23}$ as vertexes. In this case, side lengths of the triangle $\Delta W_{21}W_{22}W_{23}$ are respectively a side length

$$W_{21}W_{22}=\text{a}=\sqrt{(20-(-20))^2+(-20-0)^2}=\sqrt{2000}$$ , a side length

$$W_{21}W_{23}=\text{b}=\sqrt{(0-(-20))^2+(20-0)^2}=\sqrt{800}$$ , and a side length

$$W_{22}W_{23}=\text{c}=\sqrt{(0-20)^2+(20-(-20))^2}=\sqrt{2000}$$ .

The wearable device may obtain, based on the cosine theorem, an angle that uses the element $W_{22}$ located in the area 4 as a vertex:

$$\theta_{645}=\arccos\frac{\text{b}^2+\text{a}^2-\text{c}^2}{2*\text{b}*\text{a}}=\arccos\frac{(W_{22}W_{23})^2+(W_{21}W_{22})^2-(W_{21}W_{23})^2}{2*W_{22}W_{23}*W_{21}W_{22}}$$
$$=\arccos\frac{(\sqrt{2000})^2+(\sqrt{2000})^2-(\sqrt{800})^2}{2*\sqrt{2000}*\sqrt{2000}}$$ .

If there are a plurality of such angles in the coordinate set **W,** the wearable device may calculate a standard deviation of these angles to obtain the feature 3. It may be understood that an existing calculation formula is used to calculate the standard deviation, and details are not described herein.

The wearable device may further calculate an angle corresponding to a slope formed by two adjacent elements in the coordinate set **W** that are respectively located in the area 2 and the area 4. If there are a plurality of such angles in the coordinate set **W,** the wearable device may calculate a modulus of these angles to obtain a feature 4, and may further calculate a standard deviation of these angles to obtain a feature 5. A location, in the coordinate set **W,** of the element located in the area 2 is in front of a location, in the coordinate set **W,** of the element located in the area 4. The feature 4 may be represented by a symbol $\theta_{24}$, and the feature 5 may be represented by a symbol $sd_{24}$, but this embodiment of this application is not limited thereto. The wearable device may further form a straight line by using two adjacent elements in the coordinate set **W** that are respectively located in the area 2 and the area 4, and calculate a distance from the origin (0, 0) to the straight line. If there are a plurality of such distances, the wearable device may calculate a standard deviation of these distances to obtain a feature 6. The feature 6 may be represented by a symbol $sd\_d_{24}$.

For example, if in the coordinate set **W,** $W_2=(x_1, y_1)=(-60, 60)$ is located in the area 2 in the Poincare plot, and $W_3=(x_2, y_2)=(20, -20)$ is located in the area 4 in the Poincare plot, the wearable device may extract the feature 4 based on $W_2$ and $W_3$. A specific extraction manner may be shown in FIG. 5. The wearable device may form a line segment $W_2W_3$ by connecting $W_2$ and $W_3$. A slope of the line segment $W_2W_3$ is $K=(y_2-y_1)/(x_2-x_1)=((-20)-60)/(20-(-60))=1$.

The wearable device may obtain the feature 4 based on the slope, that is, $\theta_{24}=arctanK=arctan1=45°$. If there are a plurality of groups of elements meeting the condition in the coordinate set **W,** the wearable device may obtain a plurality of angle values based on the method shown in FIG. 5, and the wearable device may calculate a standard deviation of the plurality of angle values to obtain the feature 5.

The wearable device may further calculate a distance from the origin (0, 0) to a straight line on which the line segment $W_2W_3$ is located, to obtain the feature 6. As shown in FIG. 5, the origin is on the line segment $W_2W_3$, and the feature 6 is 0, that is, $sd\_d_{24}=0$.

The wearable device may further calculate an angle corresponding to a slope formed by two adjacent elements in the coordinate set **W** that are respectively located in the area 4 and the area 2. If there are a plurality of such angles in the coordinate set **W,** the wearable device may calculate a modulus of these angles to obtain a feature 7, and may further calculate a standard deviation of these angles to obtain a feature 8. A location, in the coordinate set **W,** of the element located in the area 4 is in front of a location, in the coordinate set **W,** of the element located in the area 2. The feature 7 may be represented by a symbol $\theta_{42}$, and the feature 8 may be represented by a symbol $sd_{42}$, but this embodiment of this application is not limited thereto. The wearable device may further form a straight line by using two adjacent elements in the coordinate set **W** that are respectively located in the area 4 and the area 2, and calculate a distance from the origin (0, 0) to the straight line. If there are a plurality of such distances, the wearable device may calculate a standard deviation of these distances to obtain a feature 9. The feature 9 may be represented by a symbol $sd\_d_{42}$.

For example, if in the coordinate set **W,** $W_3=(x_2, y_2)=(20, -20)$ is located in the area 4 in the Poincare plot, and $W_4=(x_1, y_1)=(-60, 60)$ is located in the area 2 in the Poincare plot, the wearable device may extract the feature 7 based on $W_3$ and $W_4$. A specific extraction manner is the same as the method shown in FIG. 5, and details are not described herein again. If there are a plurality of groups of elements meeting the condition in the coordinate set **W,** the wearable device may obtain a plurality of angle values based on the method shown in FIG. 5, and the wearable device may calculate a standard deviation of the plurality of angle values to obtain the feature 8. The wearable device may further calculate a distance from the origin (0, 0) to a straight line on which $W_3$ and $W_4$ are located, to obtain the feature 9.

The statistical feature includes the plurality of features, and has a plurality of possible implementations.

In a possible example, the statistical feature includes the feature 1 ($r_{zero}$), the feature 2 ($sd_{123}$), the feature 3 ($sd_{645}$), the feature 6 ($sd\_d_{24}$), and the feature 9 ($sd\_d_{42}$). The wearable device may identify, based on the feature 1, the feature 2, the feature 3, the feature 6, and the feature 9, whether a heart rhythm is a normal heart rhythm or an abnormal heart rhythm. If the heart rhythm is an abnormal heart rhythm, the wearable device may further identify whether the abnormal heart rhythm is atrial fibrillation or premature beat.

In another possible example, the statistical feature includes the feature 1 ($r_{zero}$), the feature 2 ($sd_{123}$), the feature 3 ($sd_{645}$), the feature 4 ($\theta_{24}$), the feature 6 ($sd\_d_{24}$), the feature 7 ($\theta_{42}$), and the feature 9 ($sd\_d_{42}$). The wearable device may identify, based on the feature 1, the feature 2, the feature 3, the feature 4, the feature 6, the feature 7, and the feature 9, whether a heart rhythm is a normal heart rhythm or an abnormal heart rhythm. If the heart rhythm is an abnormal heart rhythm, the wearable device may further identify whether the abnormal heart rhythm is atrial fibrillation, premature beat, premature beat with bigeminy, or premature beat with trigeminy.

In still another possible example, the statistical feature includes the feature 1 ($r_{zero}$), the feature 2 ($sd_{123}$), the feature 3 ($sd_{645}$), the feature 5 ($sd_{24}$), the feature 6 ($sd\_d_{24}$), the feature 8 ($sd_{42}$), and the feature 9 ($sd\_d_{42}$). The wearable device may identify, based on the feature 1, the feature 2, the feature 3, the feature 5, the feature 6, the feature 8, and the feature 9, whether a heart rhythm is a normal heart rhythm or an abnormal heart rhythm. If the heart rhythm is an abnormal heart rhythm, the wearable device may further identify whether the abnormal heart rhythm is atrial fibrillation, premature beat, premature beat with bigeminy, or premature beat with trigeminy.

In another possible example, the statistical feature includes the feature 1 ($r_{zero}$), the feature 2 ($sd_{123}$), the feature

3 ($sd_{645}$), the feature 4 ($\theta_{24}$), the feature 5 ($sd_{24}$), the feature 6 ($sd\_d_{24}$), the feature 7 ($\theta_{42}$), the feature 8 ($sd_{42}$), and the feature 9 ($sd\_d_{42}$). The wearable device may identify, based on the feature 1, the feature 2, the feature 3, the feature 4, the feature 5, the feature 6, the feature 7, the feature 8, and the feature 9, whether a heart rhythm is a normal heart rhythm or an abnormal heart rhythm. If the heart rhythm is an abnormal heart rhythm, the wearable device may further identify whether the abnormal heart rhythm is atrial fibrillation, premature beat, premature beat with bigeminy, or premature beat with trigeminy.

S215. Input the statistical feature to a machine learning model, to obtain an identification result.

The machine learning model may be a model such as a random forest, a gradient boosting decision tree (gradient boosting decision tree, GBDT), extreme gradient boosting (Xgboost), or a support vector machine. This is not limited in this embodiment of this application. Training samples of the machine learning model may include a statistical feature of a PPG signal of atrial fibrillation, a statistical feature of a PPG signal of premature beat, a statistical feature of a PPG signal of a sinus rhythm, a statistical feature of a PPG signal of atrial flutter, a statistical feature of a PPG signal of atrioventricular conduction block, a statistical feature of a PPG signal of bundle branch conduction block, and the like.

The identification result has a plurality of implementations. In a possible implementation, the identification result may be that the heart rhythm is normal or the heart rhythm is abnormal. In another possible implementation, the identification result may be that the heart rhythm is normal or a type of heart rhythm abnormality.

If the statistical feature includes the feature 1, the feature 2, and the feature 3, the identification result may be a normal heart rhythm, atrial fibrillation, or premature beat. If the statistical feature includes the feature 1, the feature 2, the feature 3, the feature 4, the feature 6, the feature 7, and the feature 9, the identification result may be a normal heart rhythm, atrial fibrillation, premature beat, premature beat with bigeminy, or premature beat with trigeminy. If the statistical feature includes the feature 1, the feature 2, the feature 3, the feature 5, the feature 6, the feature 8, and the feature 9, the identification result may be a normal heart rhythm, atrial fibrillation, premature beat, premature beat with bigeminy, or premature beat with trigeminy. If the statistical feature includes the feature 1, the feature 2, the feature 3, the feature 4, the feature 5, the feature 6, the feature 7, the feature 8, and the feature 9, the identification result may be a normal heart rhythm, atrial fibrillation, premature beat, premature beat with bigeminy, or premature beat with trigeminy.

According to the heart rhythm abnormality identification method provided in this embodiment of this application, a statistical feature is extracted based on a PPG signal, and the statistical feature is input to a machine learning model, to obtain an identification result. Compared with using an average heart rate to represent an abnormal heart rhythm, using the statistical feature to represent an abnormal heart rhythm helps improve accuracy of heart rhythm abnormality identification. Compared with a method of determining based on a threshold, using the machine learning model to identify an abnormal heart rhythm helps improve accuracy and flexibility of identification.

In an optional embodiment, after S210 of extracting IBIs based on the peak value set, to obtain an IBI set, and before S215 of inputting the statistical feature to a machine learning model, to obtain an identification result, the method 200 may further include: extracting at least one of a time-domain feature, a frequency-domain feature, or a non-linear feature based on elements in the IBI set. S215 may include a plurality of cases: If the wearable device extracts the time-domain feature, the statistical feature and the time-domain feature are input to a machine learning model, to obtain an identification result. If the wearable device extracts the frequency-domain feature, the statistical feature and the frequency-domain feature are input to a machine learning model, to obtain an identification result. If the wearable device extracts the non-linear feature, the statistical feature and the non-linear feature are input to a machine learning model, to obtain an identification result. If the wearable device extracts the time-domain feature and the frequency-domain feature, the statistical feature, the time-domain feature, and the frequency-domain feature are input to a machine learning model, to obtain an identification result. If the wearable device extracts the time-domain feature and the non-linear feature, the statistical feature, the time-domain feature, and the non-linear feature are input to a machine learning model, to obtain an identification result. If the wearable device extracts the frequency-domain feature and the non-linear feature, the statistical feature, the frequency-domain feature, and the non-linear feature are input to a machine learning model, to obtain an identification result. Alternatively, if the wearable device extracts the time-domain feature, the frequency-domain feature, and the non-linear feature, the statistical feature, the time-domain feature, the frequency-domain feature, and the non-linear feature are input to a machine learning model, to obtain an identification result.

In this embodiment of this application, a time-domain feature may be extracted in a time-domain dimension based on the elements in the IBI set ($\mathbf{TP}=[TP_1, TP_2, ..., TP_{q-1}]$). The time-domain feature may include at least one of a mean value (which may be represented by a symbol IBI_mean) of the elements in the IBI set ($\mathbf{TP}=[TP_1, TP_2, ..., TP_{q-1}]$), a median (which may be represented by a symbol IBI_median) of the elements in the IBI set, a standard deviation (which may be represented by a symbol SDNN) of the elements in the IBI set, a root mean square (which may be represented by a symbol RMSSD) of differences between adjacent elements in the IBI set, a standard deviation (which may be represented by a symbol SDSD) of the differences between the adjacent elements in the IBI set, or a ratio (which may be represented by a symbol pNN50) of a quantity of differences greater than 50 milliseconds between adjacent elements to a total quantity of elements in the IBI set.

For example, the mean value of the elements in the IBI set ($\mathbf{TP}=[TP_1, TP_2, ..., TP_{q-1}]$) is IBI_mean, and a calculation formula of IBI_mean is as follows:

$$IBI\_mean = \frac{TP_1 + TP_2 + ... + TP_{q-1}}{q-1}.$$

The standard deviation of the elements in the IBI set (**TP**=[$TP_1$, $TP_2$, ..., $TP_{q-1}$]) is SDNN, and a calculation formula of SDNN is as follows:

$$SDNN = \sqrt{\frac{(TP_1 - IBI\_mean)^2 + (TP_2 - IBI\_mean)^2 + ... + (TP_{q-1} - IBI\_mean)^2}{q-1}}.$$

The root mean square of the differences between the adjacent elements in the IBI set (**TP**=[$TP_1$, $TP_2$, ..., $TP_{q-1}$]) is RMSSD, and a calculation formula of RMSSD is as follows:
If a set of the differences between the adjacent elements in the IBI set may be represented by ΔTP, **ΔTP**=[$\Delta TP_1$, $\Delta TP_2$, ..., $\Delta TP_{p-2}$]. $\Delta TP_1 = TP_2 - TP_1$, $\Delta TP_2 = TP_3 - TP_2$, ..., and $\Delta TP_{q-2} = TP_{q-1} - TP_{q-2}$. In this case,

$$RMSSD = \sqrt{\frac{(\Delta TP_1)^2 + (\Delta TP_2)^2 + ... + (\Delta TP_{q-2})^2}{q-2}}.$$

The standard deviation of the differences between the adjacent elements in the IBI set (**TP**=[$TP_1$, $TP_2$, ..., $TP_{q-1}$]) is SDSD, and calculation formulas of SDSD are as follows:

$$\Delta TP\_mean = \frac{\Delta TP_1 + \Delta TP_2 + ... + \Delta TP_{q-2}}{q-1};$$

and

$$SDSD = \sqrt{\frac{(\Delta TP_1 - \Delta TP\_mean)^2 + (\Delta TP_2 - \Delta TP\_mean)^2 + ... + (\Delta TP_{q-2} - \Delta TP\_mean)^2}{q-2}}.$$

In an example, the time-domain feature may include IBI_mean, SDNN, and SDSD.
In another example, the time-domain feature may include IBI_mean, IBI_median, SDNN, RMSSD, SDSD, and pNN50.
In this embodiment of this application, a frequency-domain feature may be further extracted in a frequency-domain dimension based on the elements in the IBI set (**TP**=[$TP_1$, $TP_2$, ..., $TP_{q-1}$]). A frequency-domain analysis method in this embodiment of this application is performing a fast fourier transform (fast fourier transform, FFT) or autoregressive (autoregressive, AR) parameter modeling operation on the elements in the IBI set, to obtain, for analysis, a power spectrum chart that uses a frequency (whose unit may be Hertz) as a horizontal coordinate and uses a power spectrum density as a vertical coordinate.
The wearable device may generate a power spectrum chart based on the elements in the IBI set, and extract the frequency-domain feature based on the power spectrum chart. The frequency-domain feature includes ultra low frequency (very low frequency, ULF) power, low frequency (low frequency, LF) power, high frequency (high frequency, HF) power, or a ratio (LF/HF) of the low frequency power to the high frequency power. The VLF is ultra low frequency power, which is power in a frequency band of 0.003 to 0.04 Hertz. The LF is low frequency power, which is power in a frequency band of 0.04 to 0.15 Hertz. The HF is high frequency power, which is power in a frequency band of 0.15 to 0.4 Hertz. The LF/HF is a ratio of the low frequency power to the high frequency power. The VLF may reflect a long-term rhythm of the user, and may be used as a marker of sympathetic activity. The LF may reflect responsiveness of a sinoatrial node to sympathetic nerve input, and is jointly regulated by sympathetic and parasympathetic nerves. The HF may reflect a breathing frequency of the user, and may be used as a marker of vagal nerve regulation.
In an example, the frequency-domain feature may include the ULF power, the HF power, and the LF power.
In another example, the frequency-domain feature may include the ULF power and the LF/HF.
In still another example, the frequency-domain feature may include the ULF power, the HF power, the LF power, and the LF/HF.

In this embodiment of this application, a non-linear feature may be further extracted in a non-linear dimension based on the elements in the IBI set (**TP**=[$TP_1$, $TP_2$, ..., $TP_{q-1}$]). The non-linear feature may include a sample entropy and/or a Shannon entropy, but this embodiment of this application is not limited thereto.

In an example, the non-linear feature may include the sample entropy. In another example, the non-linear feature may include the sample entropy and the Shannon entropy.

Steps of calculating the Shannon entropy may include:

1) The terminal device determines a maximum value *TPmax* and a minimum value *TPmin* from the IBI set, and calculate a difference (*TPmax-TPmin*) between the maximum value *TPmax* and the minimum value *TPmin*.

2) The terminal device divides the difference (*TPmax-TPmin*) into N equal parts to obtain N areas: $S_1$, $S_2$, ..., and $S_N$, which may be denoted as S={$S_1$,$S_2$,..., $S_N$}. N is an integer greater than or equal to 2.

3) The terminal device calculates a quantity of elements falling into each of the N areas in the elements in the IBI set, and calculates a ratio of the quantity of elements in each area to the total quantity (that is, q-1) of elements, to obtain a probability that the elements in the IBI set fall into each area. An area $S_i$ is any one of the N areas, and a probability that the elements in the IBI set fall into the area is $S_i$ is $p(S_i)$, where $1 \le i \le N$.

4) The terminal device calculates the Shannon entropy $H(S_i)$ by using the following formula:

$$H(\mathrm{S}) = -\sum\nolimits_{1}^{N} p(S_i)\log_2 p(S_i).$$

Steps of calculating the sample entropy may include:

1) The terminal device constructs, based on **TP**=[$TP_1$, $TP_2$, ..., $TP_{q-1}$]), a vector sequence in an m-dimensional space: X(1), X(2), ..., and X((q-1)-m+1), where X($i$)={$TP_i$,$TP_{i+1}$,...,$TP_{i+m}$}, $1 \le i \le$ q - m, and q-m=q-1-m+1.

2) Define a distance d[X($i$),X($j$)] between vectors X($i$) and X($j$) to be a maximum difference between elements corresponding to the two vectors, that is, $\mathrm{d}\left[ \mathrm{X}\left(i\right), \mathrm{X}\left(j\right)\right] = \max\limits_{\mathrm{k}=0.1...,\mathrm{m}-1}\left|TP_{i+\mathrm{k}} - TP_{j+\mathrm{k}}\right|$, where $j$ is different from $i$, and $j$ is an integer greater than or equal to 1 and less than or equal to q-m (that is, q-1-m+1).

3) For each $i$, when a tolerance deviation is r, a quantity of d[X($i$),X($j$)] < r is counted, and denoted as $B_i$, and a ratio $B_i^m(r)$ of the data to a total quantity of distances is calculated, where $B_i^m(r)$ may be expressed as follows:

$$B_i^{\,m}(r) = \frac{1}{(q-1)-\mathrm{m}-1} B_i$$

4) $B_i^m(r)$ is defined as follows:

$$B_i^{\,m}(r) = \frac{1}{(q-1)-\mathrm{m}} \sum_{i=1}^{(p-1)-m} B_i^{\,m}(r)$$

5) Increase the quantity of dimensions to m+1, and repeat the foregoing steps 1) to 4) to obtain $A_i$, where $A_i$ may be expressed as follows:

$$A^{m}(r) = \frac{1}{(q-1)-\mathrm{m}} \sum_{i=1}^{(p-1)-m} A_i^{\,m}(r)$$

In this way, $B_i^m(r)$ is a probability that two sequences match m points with the tolerance deviation of r, and $A^m(r)$ is a probability that two sequences match m+1 points with the tolerance deviation of r. The terminal device calculates the sample entropy by using the following formula:

$$\mathrm{SampEn(m,r,q-1)} = -\ln\left[\frac{A^m(r)}{B^m(r)}\right].$$

If the wearable device further extracts the time-domain feature, the frequency-domain feature, and the non-linear feature, in addition to the statistical feature, the statistical feature, the time-domain feature, the frequency-domain feature, and the non-linear feature are input to a machine learning model, to obtain an identification result.

For example, FIG. 6 is a block diagram of heart rhythm abnormality identification. After extracting the statistical feature, the time-domain feature, the frequency-domain feature, and the non-linear feature, the wearable device uses the statistical feature, the time-domain feature, the frequency-domain feature, and the non-linear feature as inputs, and inputs them to a machine learning model, to obtain an output of the machine learning model, that is, an identification result. The identification result may be a sinus rhythm, premature beat, atrial fibrillation, premature beat with bigeminy, or premature beat with trigeminy.

According to the heart rhythm abnormality identification method provided in this embodiment of this application, the statistical feature is used in combination with at least one of the time-domain feature, the frequency-domain feature, or the non-linear feature to perform heart rhythm abnormality identification, so that features are extracted in a plurality of dimensions, which helps represent an abnormal heart rhythm more accurately, thereby improving accuracy of heart rhythm abnormality identification.

In an optional embodiment, before S201 of obtaining acceleration data by using an Acc, and collecting a PPG signal, the method 200 may further include: The wearable device obtains acceleration data within a preset duration C by using the Acc, and determines, based on the acceleration data within the preset duration C, whether the wearable device keeps in the static state within the preset duration C. If the wearable device keeps in the static state within the preset duration C, the wearable device may perform S201. If the wearable device does not keep in the static state within the preset duration C, or the wearable device is not in the static state at one or more moments within the preset duration C, the wearable device may exit heart rhythm detection.

The preset duration C may be less than the preset duration A. The preset duration C may be greater than, less than, or equal to the preset duration B. This is not limited in this embodiment of this application.

There are a plurality of implementations in which the wearable device obtains the acceleration data within the preset duration C by using the Acc, and determines, based on the acceleration data within the preset duration C, whether the wearable device keeps in the static state within the preset duration C.

In a possible implementation, the wearable device may obtain acceleration data within a duration 1. The duration 1 is less than the preset duration C. The wearable device may determine whether the wearable device keeps in the static state within the duration 1. If the wearable device is in the static state, and the duration 1 is less than the preset duration C, the wearable device continues to obtain acceleration data within a duration 2. The duration 2 may or may not be equal to the duration 1. This is not limited in this embodiment of this application. The wearable device may determine whether the wearable device keeps in the static state within the duration 2. If the wearable device is in the static state within the duration 2, and a sum of the duration 1 and the duration 2 (that is, the acceleration obtaining duration) is still less than the preset duration C, the wearable device continues to obtain acceleration data until the acceleration obtaining duration is greater than or equal to the preset duration C, or the wearable device exits heart rhythm detection when the wearable device is in the non-static state.

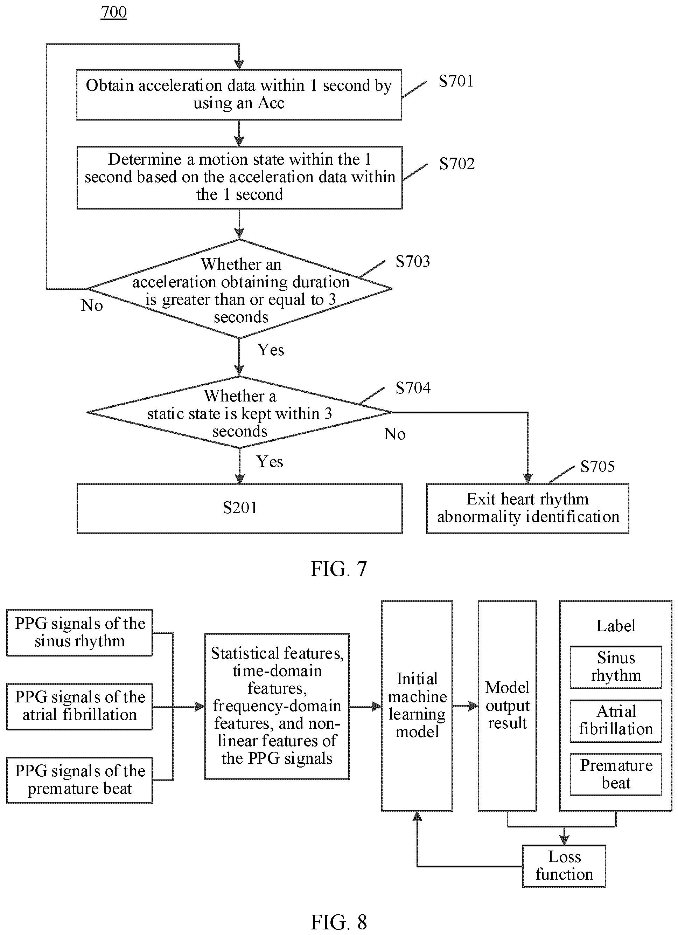

For example, the duration 1 is equal to the duration 2, which are equal to 1 second. The preset duration C is equal to 3 seconds. FIG. 7 is a schematic flowchart of a heart rhythm abnormality identification method 700. As shown in FIG. 7, the method 700 may include the following steps.

S701. Obtain acceleration data within 1 second by using an Acc.

There may be a plurality of trigger conditions of S701. In a possible example, a trigger condition of S701 is that a wearable device detects an operation of a user triggering a heart rhythm detection option. To be specific, the wearable device detects the operation of the user triggering the heart rhythm detection option, and performs S701 in response to the operation of the user triggering the heart rhythm detection option. In another possible example, a trigger condition of S701 is that the wearable device detects that a heart rhythm detection time arrives. The wearable device may provide a function of periodic heart rhythm detection or regular heart rhythm detection for the user. When the wearable device detects that the function of periodic heart rhythm detection or regular heart rhythm detection is enabled, the wearable device may monitor, in real time, whether the heart rhythm detection time arrives. When the wearable device detects that the heart rhythm detection time arrives, S701 is performed.

The Acc may be the acceleration sensor 180E in FIG. 1, but this embodiment of this application is not limited thereto. The wearable device may obtain acceleration data of the wearable device on an X axis, a Y axis, and a Z axis by using the Acc. If the wearable device obtains, by using the Acc, acceleration data $x_0$ of the wearable device on the x axis, acceleration data $y_0$ on the Y axis, and acceleration data $z_0$ on the z axis, the acceleration data obtained by the wearable device by using the Acc is $(x_0, y_0, z_0)$.

The wearable device obtains acceleration data within 1 second, and the wearable device may determine a modulus of all acceleration data on the three axes within the 1 second as the acceleration data of the wearable device within the 1 second.

S702. Determine a motion state of the wearable device within the 1 second based on the acceleration data within the 1 second.

The motion state includes a static state and a non-static state. The wearable device may compare the acceleration data within the 1 second with an acceleration threshold. If the acceleration data is less than or equal to the acceleration threshold, it is determined that the wearable device is in the static state. If the acceleration data is greater than the acceleration threshold, it is determined that the wearable device is in the non-static state.

S703. Determine whether an acceleration obtaining duration is greater than or equal to 3 seconds.

The acceleration obtaining duration is a duration within which the wearable device obtains acceleration data by using the Acc. If the acceleration obtaining duration is less than 3 seconds, the wearable device may continue to obtain acceleration data within 1 second by using the Acc, and determine a motion state within the 1 second based on the acceleration data within the 1 second, that is, perform S701 and S702. If the acceleration obtaining duration is greater than or equal to 3 seconds, the wearable device may determine whether the wearable device keeps in the static state within 3 consecutive seconds.

For example, if the acceleration obtaining duration is 1 second, as the acceleration obtaining duration is less than 3 seconds, the wearable device may continue to obtain acceleration data within 1 second by using the Acc. Then, the acceleration obtaining duration is 1+1=2 seconds. As the acceleration obtaining duration is less than 3 seconds, the wearable device may continue to obtain acceleration data within 1 second by using the Acc. Then, the acceleration obtaining duration is 2+1 =3 seconds. As the acceleration obtaining duration is equal to 3 seconds, the wearable device determines whether the acceleration data keeps in the static state within the 3 seconds.

S704. If the acceleration obtaining duration is greater than or equal to 3 seconds, determine whether the wearable device keeps in the static state within 3 seconds.

If the wearable device keeps in the static state within 3 seconds, the wearable device obtains acceleration data by using the Acc, and collects a PPG signal, that is, performs S201 in the method 200. If the wearable device does not keep in the static state within 3 seconds, it may indicate that the wearable device does not meet a heart rhythm abnormality identification condition at this time, and the wearable device may exit heart rhythm abnormality identification, that is, perform S705.

S705. If the wearable device does not keep in the static state within 3 seconds, exit heart rhythm abnormality identification.

That the wearable device exits heart rhythm abnormality identification means skipping heart rhythm abnormality identification. After exiting heart rhythm abnormality identification, the wearable device may further output prompt information to prompt the user that the wearable device is in the non-static state and recommend the user to keep the wearable device static. The wearable device may further clear the collected acceleration data to save internal memory space.

In this implementation, acceleration data is obtained in a small time unit, and a motion state of the wearable device is determined. When the motion state is the non-static state, heart rhythm abnormality identification may be directly exited. The acceleration data within the preset duration C does not need to be completely obtained, which helps improve efficiency of heart rhythm abnormality identification.

In another possible implementation, the wearable device may continuously obtain the acceleration data within the preset duration C by using the Acc, and determine, based on the acceleration data within the preset duration C, whether the wearable device keeps in the static state within the preset duration C.

For example, the preset duration C may be 3 seconds. The wearable device may obtain acceleration data of the wearable device on an X axis, a Y axis, and a Z axis within 3 seconds by using the Acc, and determine a modulus of all the acceleration data on the three axes within the 3 seconds as the acceleration data of the wearable device within the 3 seconds. The wearable device may compare the acceleration data within the 3 second with an acceleration threshold. If the acceleration data is less than or equal to the acceleration threshold, it is determined that the wearable device is in the static state. If the acceleration data is greater than the acceleration threshold, it is determined that the wearable device is in the non-static state.

In this implementation, the acceleration data within the preset duration C is directly collected, and a motion state of the wearable device within the preset duration C is determined based on the acceleration data within the preset duration C, which is simple and convenient.

According to the heart rhythm abnormality identification method provided in this embodiment of this application, before heart rhythm abnormality identification, whether the wearable device keeps in the static state within the preset duration C may be determined first. If the wearable device keeps in the static state within the preset duration C, it may indicate that the heart rhythm abnormality identification condition is met, and heart rhythm abnormality identification may be performed. If the wearable device does not keep in the static state within the preset duration C, it may indicate that the heart rhythm abnormality identification condition is not met, and heart rhythm abnormality identification may not be performed. In this manner, heart rhythm abnormality identification is performed when the heart rhythm abnormality identification condition is met, which helps ensure accuracy of heart rhythm abnormality identification.

A method of using a machine learning model is described in detail above, and a method of training a machine learning model is described in detail below.

The machine learning model may be trained by a processing device having a processing function, including but not limited to a wearable device. For example, the processing device may be a computer, a mobile phone, a smart watch, or a smart band. In this embodiment of this application, an example that the processing device is a wearable device is used for description.

The machine learning model may be configured to identify a normal heart rhythm (that is, a sinus rhythm) and an abnormal heart rhythm. The abnormal heart rhythm may include at least one of atrial fibrillation, premature beat, atrial flutter, atrioventricular conduction block, and bundle branch conduction block. In this embodiment of this application, the method of training a machine learning model is described by using an example that the machine learning model is configured to identify a sinus rhythm, atrial fibrillation, and premature beat.

FIG. 8 is a block diagram of machine model training. As shown in FIG. 8, the wearable device may obtain a plurality of PPG signals of the sinus rhythm, a plurality of PPG signals of the atrial fibrillation, and a plurality of PPG signals of the premature beat. In this embodiment of this application, quantities of the PPG signals and a way in which the wearable device obtains the PPG signals are not limited.

A waveform in each periodicity of the PPG signal of the sinus rhythm is regular. Waveforms in periodicities of the PPG signal of the atrial fibrillation and the PPG signal of the premature beat are irregular. The PPG signal of the atrial fibrillation may include a PPG signal of normal atrial fibrillation and a PPG signal of rapid atrial fibrillation. The PPG signal of the premature beat may include a PPG signal of normal premature beat, a PPG signal of premature beat with bigeminy, and a PPG signal of premature beat with trigeminy.

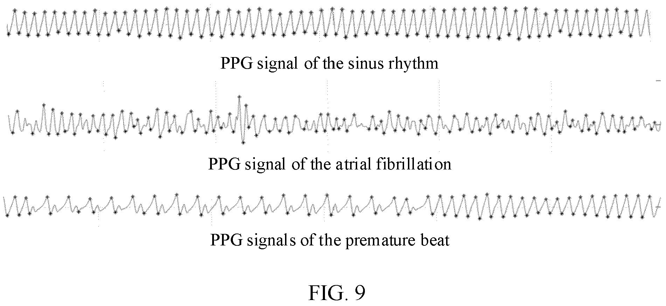

For example, FIG. 9 is a diagram of PPG signals of different heart rhythms. As shown in FIG. 9, a peak and a trough exist in each periodicity of a PPG signal of a sinus rhythm, the peak and the trough are both represented by black signs, a difference between waveforms in different periodicities is small, and fluctuation is regular. A peak and a trough exist in each periodicity of a PPG signal of (normal) atrial fibrillation, the peak and the trough are both represented by black signs, a difference between waveforms in different periodicities is large, and fluctuation is irregular. A peak and a trough exist in each periodicity of a PPG signal of a (normal) premature beat, the peak and the trough are both represented by black signs, and a waveform changes in stages.

The wearable device may extract a statistical feature, a time-domain feature, a frequency-domain feature, and a non-linear feature of each PPG signal. The statistical feature may include a feature 1 ($r_{zero}$), a feature 2 ($sd_{123}$), a feature 3 ($sd_{645}$), a feature 4 ($\theta_{24}$), a feature 5 ($sd_{24}$), a feature 6 ($sd\_d_{24}$), a feature 7 ($\theta_{42}$), a feature 8 ($sd_{42}$), and a feature 9 ($sd\_d_{42}$).

For example, the wearable device may preprocess each PPG signal to obtain a preprocessed PPG signal, extract peak value data in the preprocessed PPG signal to obtain a peak value set corresponding to each PPG signal, and extract IBIs based on the peak value set to obtain an IBI set. The wearable device may obtain a heart rate set based on a heart rate corresponding to each element in the IBI set, calculate differences between adjacent heart rates based on the heart rate set to obtain a difference set, then construct coordinates based on adjacent elements in the difference set to obtain a coordinate set, and finally extract the statistical feature based on areas, in a Poincare plot, of elements in the coordinate set. For specific steps, refer to S210 to S214 in the method 200. Details are not described herein again.

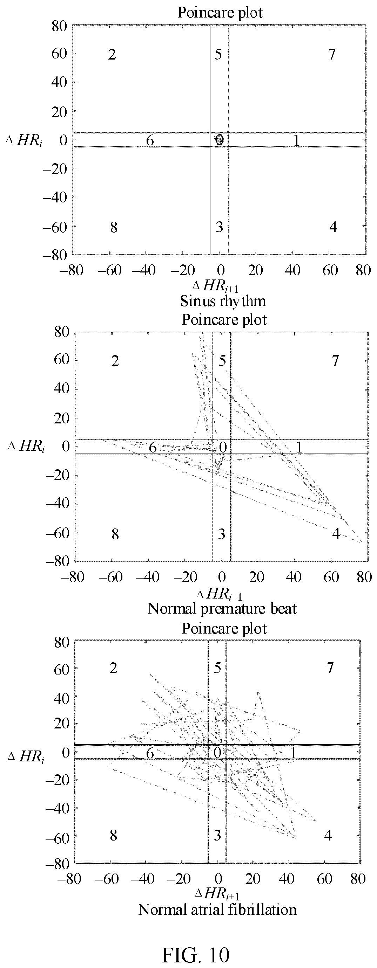

Elements in coordinate sets corresponding to different PPG signals have different area distribution in the Poincare plot. FIG. 10 is a diagram of area distribution, in a Poincare plot, of elements in coordinate sets corresponding to different PPG signals. As shown in FIG. 10, areas, in the Poincare plot, of all elements in a coordinate set corresponding to the PPG signal of the sinus rhythm are an area 0. Therefore, in features of the PPG signal of the sinus rhythm, $r_{zero}$ is a ratio of a quantity of elements located in the area 0 to a total quantity of elements in the coordinate set, and $r_{zero}1$ has a large value, and may be 1; $sd_{123}$ is an angle that is formed by three adjacent elements in the coordinate set respectively located in an area 1, an area 2, and an area 3 and that uses the element located in the area 2 as a vertex; and $sd_{645}$ is an angle that is formed by three adjacent elements in the coordinate set respectively located in an area 6, an area 4, and an area 5 and that uses the element located in the area 4 as a vertex. As no elements in the coordinate set meet the conditions, the angles $sd_{123}$ and $sd_{645}$ may be 0.

An area distribution trajectory, in the Poincare plot, of elements in a coordinate set corresponding to the PPG signal of the normal premature beat is like a kite, and trajectories formed by many elements overlap. In features of the PPG signal of the normal premature beat, angles $sd_{123}$ and $sd_{645}$ are small. Area distribution, in the Poincare plot, of elements in a coordinate set corresponding to the PPG signal of the normal atrial fibrillation is random, and trajectories formed by different elements basically do not overlap. Therefore, in features of the PPG signal of the normal atrial fibrillation, angles $sd_{123}$ and $sd_{645}$ are large. The wearable device may train the machine learning model based on the feature 1 ($r_{zero}$), the feature 2 ($sd_{123}$), and the feature 3 ($sd_{645}$) to improve accuracy of heart rhythm abnormality identification.

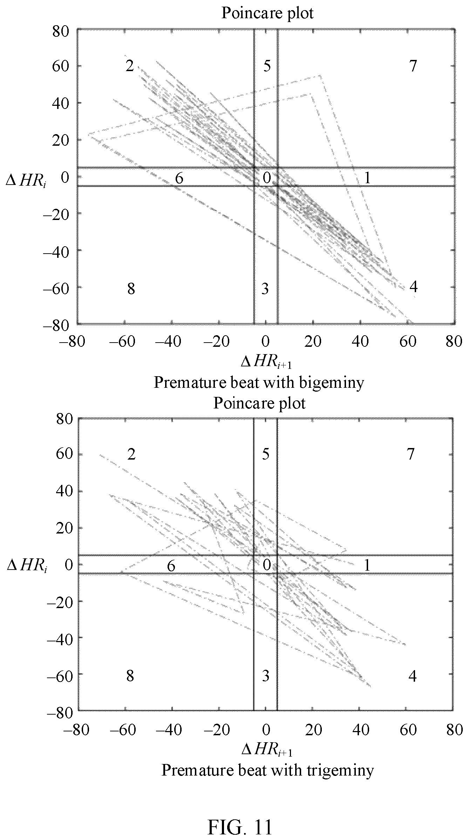

The signal of the premature beat further includes the PPG signal of the premature beat with bigeminy and the PPG signal of the premature beat with trigeminy. Area distribution, in the Poincare plot, of elements in a coordinate set corresponding to the PPG signal of the premature beat with bigeminy is different from that of the PPG signal of the premature beat with trigeminy. FIG. 11 is a diagram of area distribution, in a Poincare plot, of elements in coordinate sets corresponding to PPG signals of different premature beat. As shown in FIG. 11, area distribution trajectories, in the Poincare plot, of elements in a coordinate set corresponding to the PPG signal of the premature beat with bigeminy overlap a lot, and are mainly concentrated in an area 2 and an area 4. Area distribution trajectories, in the Poincare plot, of elements in a coordinate set corresponding to the PPG signal of the premature beat with trigeminy also overlap a lot, and are also mainly concentrated in the area 2 and the area 4. The wearable device may extract features 4 ($\theta_{24}$), features 5 ($sd_{24}$), features 6 ($sd\_d_{24}$), features 7 ($\theta_{42}$), features 8 ($sd_{42}$), and features 9 ($sd\_d_{42}$) of the PPG signal of the premature beat with bigeminy and the PPG signal of the premature beat with trigeminy to train the machine learning model, so as to improve accuracy of heart rhythm abnormality identification.

The time-domain feature may include a mean value (IBI_mean) of the elements in the IBI set, a median (IBI_median) of the elements in the IBI set, a standard deviation (SDNN) of the elements in the IBI set, a root mean square (RMSSD) of differences between adjacent elements in the IBI set, a standard deviation (SDSD) of the differences between the adjacent elements in the IBI set, or a ratio (pNN50) of a quantity of differences greater than 50 milliseconds between adjacent elements to a total quantity of elements in the IBI set. The frequency-domain feature may include ultra low frequency (ULF) power, low frequency (LF) power, high frequency (HF) power, and a ratio of the low frequency power to the high frequency power. The non-linear feature may include a sample entropy and a Shannon entropy.

The wearable device may use the extracted features of each PPG signal as inputs of an initial machine learning model, to obtain a model output result, compare the model output result with a label (a sinus rhythm, atrial fibrillation, or premature beat) corresponding to each signal, calculate a loss function, and continuously correct a parameter of the initial machine learning model by using the loss function, to obtain a machine learning model.

For example, the wearable device may use features of the plurality of PPG signals of the sinus rhythm as inputs of the initial machine learning model, to obtain a model output result, compare the model output result with the sinus rhythm label, calculate the loss function, and continuously correct the parameter of the initial machine learning model by using the loss function; use features of the plurality of PPG signals of the atrial fibrillation as inputs of the initial machine learning model, to obtain a model output result, compare the model output result with the atrial fibrillation label, calculate the loss function, and continuously correct the parameter of the initial machine learning model by using the loss function; and use features of the plurality of PPG signals of the premature beat as inputs of the initial machine learning model, to obtain a model output result, compare the model output result with the premature beat label, calculate the loss function, and continuously correct the parameter of the initial machine learning model by using the loss function, to finally obtain the machine learning model.

The wearable device may further verify robustness of the machine learning model by using test samples.

For example, the wearable device may extract statistical features, time-domain features, frequency-domain features, and non-linear features of 100 PPG signals of the sinus rhythm, 100 PPG signals of the atrial fibrillation, and 100 PPG signals of the premature beat, and input the statistical features, the time-domain features, the frequency-domain features, and the non-linear features of the PPG signals to the machine learning model, to obtain an identification result of each PPG signal. If the identification result of the PPG signal is the same as a label, the identification result is marked as correct. If the identification result of the PPG signal is different from the label, the identification result is marked as incorrect. The wearable device may determine a ratio of a quantity of correct marks in the 100 PPG signals of the sinus rhythm to 100 as accuracy of identifying the sinus rhythm, may determine a ratio of a quantity of correct marks in the 100 PPG signals of the atrial fibrillation to 100 as accuracy of identifying the atrial fibrillation, and may determine a ratio of a quantity of correct marks in the 100 PPG signals of the premature beat to 100 as accuracy of identifying the premature beat. The wearable device may further calculate overall accuracy of the machine learning model. Specifically, the wearable device may calculate a sum of the quantity of correct marks in the 100 PPG signals of the sinus rhythm, the quantity of correct marks in the 100 PPG signals of the atrial fibrillation, and the quantity of correct marks in the 100 PPG signals of the premature beat, and calculate a ratio of the sum to a total quantity (0+100+100=300), to obtain the overall accuracy. Higher overall accuracy indicates better robustness.

Sequence numbers of processes in the foregoing embodiments do not mean a specific execution sequence. The execution sequences of the processes should be determined based on functions and internal logic of the processes and should not be construed as any limitation on implementation processes of embodiments of this application.

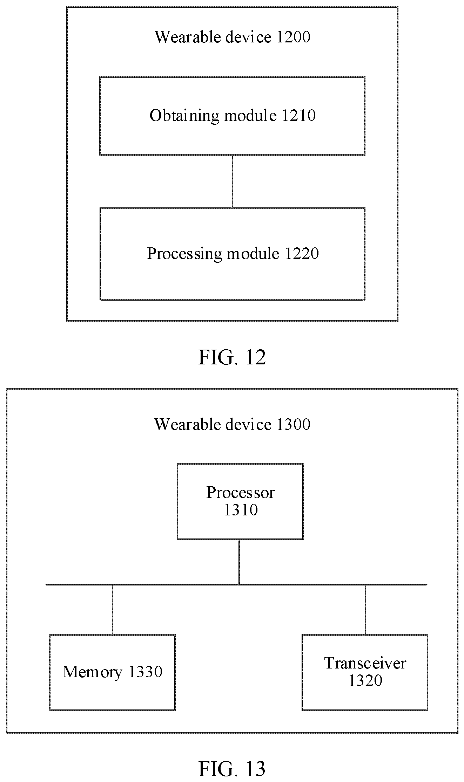

The method provided in embodiments of this application is described in detail above with reference to FIG. 1 to FIG. 11. The wearable device provided in embodiments of this application is described in detail below with reference to FIG. 12 and FIG. 13.

FIG. 12 is a diagram of a wearable device 1200 according to an embodiment of this application. The wearable device 1200 includes: an obtaining module 1210 and a processing module 1220. The obtaining module 1210 is configured to obtain heart rate data of a user. The processing module 1220 is configured to: determine a target feature of the heart rate data based on the heart rate data and a Poincare plot, where the target feature includes a first feature and a second feature, the first feature is a standard deviation of a distance from an origin of the Poincare plot to a first straight line, the first straight line is a straight line formed by first heart rate data located in an area 2 in the Poincare plot and second heart rate data located in an area 4 in the Poincare plot, an obtaining time of the first heart rate data is earlier than an obtaining time of the second heart rate data, the obtaining time of the first heart rate data is adjacent to the obtaining time of the second heart rate data, the second feature is a standard deviation of a distance from the origin of the Poincare plot to a second straight line, the second straight line is a straight line formed by third heart rate data located in the area 4 in the Poincare plot and fourth heart rate data located in the area 2 in the Poincare plot, an obtaining time of the third heart rate data is earlier than an obtaining time of the fourth heart rate data, and the obtaining time of the third heart rate data is adjacent to the obtaining time of the fourth heart rate data; and input the target feature to an identification model, to obtain an identification result, where the identification model is configured to identify, based on the input, whether a heart rhythm is abnormal.

It should be understood that the wearable device 1200 herein is embodied in a form of functional modules. The term "module" herein may be an application-specific integrated circuit (application-specific integrated circuit, ASIC), an electronic circuit, a processor (for example, a shared processor, a dedicated processor, or a group processor) for executing one or more software or firmware programs and a memory, an integrated logic circuit, and/or another suitable component that supports the described function. In an optional example, a person skilled in the art may understand that the wearable device 1200 may be specifically the wearable device in the foregoing method embodiments, or functions of the wearable device in the foregoing method embodiments may be integrated in the wearable device 1200, and the wearable device 1200 may be configured to perform procedures and/or steps corresponding to the wearable device in the foregoing method embodiments. To avoid repetition, details are not described herein again.

The wearable device 1200 has functions of implementing corresponding steps performed by the wearable device in the foregoing method embodiments. The functions may be implemented by hardware, or may be implemented by hardware executing corresponding software. The hardware or the software includes one or more modules corresponding to the functions.

In this embodiment of this application, the wearable device 1200 in FIG. 12 may alternatively be a chip or a chip system, for example, a system on chip (system on chip, SoC).

FIG. 13 is a block diagram of another wearable device 1300 according to an embodiment of this application. The wearable device 1300 includes a processor 1310, a transceiver 1320, and a memory 1330. The processor 1310, the transceiver 1320, and the memory 1330 communicate with each other through an internal connection path. The memory 1330 is configured to store instructions. The processor 1310 is configured to execute the instructions stored in the memory 1330, to control the transceiver 1320 to send a signal and/or receive a signal.

It should be understood that, a person skilled in the art may understand that the wearable device 1300 may be specifically the wearable device in the foregoing method embodiments, or functions of the wearable device in the foregoing method embodiments may be integrated in the wearable device 1300, and the wearable device 1300 may be configured to perform steps and/or procedures corresponding to the wearable device in the foregoing method embodiments. Optionally, the memory 1330 may include a read-only memory and a random access memory, and provide instructions and data to the processor 1310. A part of the memory 1330 may further include a non-volatile random access memory. For example, the memory 1330 may further store information about a device type. The processor 1310 may be configured to execute the instructions stored in the memory 1330, and when the processor 1310 executes the instructions, the processor 1310 may perform steps and/or procedures corresponding to the wearable device in the foregoing method embodiments.

It should be understood that, in this embodiment of this application, the processor 1310 may be a central processing unit (central processing unit, CPU), or the processor may be a general-purpose processor, a digital signal processor (DSP), an application-specific integrated circuit (ASIC), a field programmable gate array (FPGA) or another programmable logic device, a discrete gate or a transistor logic device, a discrete hardware component, or the like. The general-purpose processor may be a microprocessor, or the processor may be any conventional processor or the like.

In an implementation process, steps in the foregoing methods may be implemented by using a hardware integrated logic circuit in the processor or instructions in a form of software. Steps of the methods disclosed with reference to embodiments of this application may be directly performed by a hardware processor, or may be performed by using a combination of hardware in the processor and a software module. The software module may be stored in a storage medium that is mature in the art, such as a random access memory, a flash memory, a read-only memory, a programmable read-only memory, an electrically erasable programmable memory, or a register. The storage medium is located in the memory. The processor executes the instructions in the memory, to complete steps of the foregoing methods in combination with hardware thereof. To avoid repetition, details are not described herein again.

This application further provides a computer-readable storage medium. The computer-readable storage medium is configured to store a computer program, and the computer program is configured to implement the method corresponding to the wearable device in the foregoing method embodiments.

This application further provides a chip system. The chip system is configured to support the wearable device in the foregoing method embodiments in implementing functions shown in embodiments of this application.

This application further provides a computer program product. The computer program product includes a computer program (which may also be referred to as code or instructions). When the computer program is run on a computer, the computer may perform the method corresponding to the wearable device in the foregoing method embodiments.

A person of ordinary skill in the art may be aware that, in combination with the examples described in the embodiments disclosed in this specification, modules and algorithm steps can be implemented by electronic hardware or a combination of computer software and electronic hardware. Whether the functions are executed in a mode of hardware or software depends on particular applications and design constraint conditions of the technical solutions. A person skilled in the art may use different methods to implement the described functions for each particular application, but it should not be considered that the implementation goes beyond the scope of this application.

It may be clearly understood by a person skilled in the art that, for convenient and brief description, for a detailed working process of the foregoing system, apparatus, and module, refer to a corresponding process in the foregoing method embodiments. Details are not described herein again.

In the several embodiments provided in this application, it should be understood that the disclosed system, apparatus, and method may be implemented in other manners. For example, the apparatus embodiment described above is merely an example. For example, the module division is merely logical function division and may be other division in actual implementation. For example, a plurality of modules or components may be combined or integrated into another system, or some features may be ignored or not performed. In addition, the displayed or discussed mutual couplings or direct couplings or communication connections may be implemented through some interfaces. The indirect couplings or communication connections between the apparatuses or modules may be implemented in electronic, mechanical, or other forms.

The modules described as separate parts may or may not be physically separate, and parts displayed as modules may or may not be physical modules, may be located in one place, or may be distributed on a plurality of network modules. Some or all of the modules may be selected according to actual needs to achieve the objectives of the solutions of the embodiments.

In addition, functional modules in embodiments of this application may be integrated into one processing module, or each module may exist alone physically, or two or more modules may be integrated into one module.

When the functions are implemented in a form of a software functional module and sold or used as an independent product, the functions may be stored in a computer-readable storage medium. Based on such an understanding, the technical solutions of this application essentially, or a part contributing to a conventional technology, or a part of the technical solutions may be implemented in a form of a software product. The computer software product is stored in a storage medium, and includes several instructions for instructing a computer device (which may be a personal computer, a server, or a network device) to perform all or a part of steps of the methods in embodiments of this application. The storage medium includes various media that can store program code, such as a USB flash drive, a removable hard disk drive, a read-only memory (read-only memory, ROM), a random access memory (random access memory, RAM), a magnetic disk, or an optical disc.

The foregoing descriptions are merely specific implementations of this application, but are not intended to limit the protection scope of embodiments of this application. Any variation or replacement readily figured out by a person skilled in the art within the technical scope disclosed in embodiments of this application shall fall within the protection scope of embodiments of this application. Therefore, the protection scope of embodiments of this application shall be subject to the protection scope of the claims.

## Claims

1.    A heart rhythm abnormality identification method, comprising:

obtaining heart rate data of a user;
determining a target feature of the heart rate data based on the heart rate data and a Poincare plot, wherein the target feature comprises a first feature and a second feature, the first feature is a standard deviation of a distance from an origin of the Poincare plot to a first straight line, the first straight line is a straight line formed by first heart rate data located in an area 2 in the Poincare plot and second heart rate data located in an area 4 in the Poincare plot, an obtaining time of the first heart rate data is earlier than an obtaining time of the second heart rate data, the obtaining time of the first heart rate data is adjacent to the obtaining time of the second heart rate data, the second feature is a standard deviation of a distance from the origin of the Poincare plot to a second straight line, the second straight line is a straight line formed by third heart rate data located in the area 4 in the Poincare plot and fourth heart rate data located in the area 2 in the Poincare plot, an obtaining time of the third heart rate data is earlier than an obtaining time of the fourth heart rate data, and the obtaining time of the third heart rate data is adjacent to the obtaining time of the fourth heart rate data; and
inputting the target feature to an identification model, to obtain an identification result, wherein the identification model is configured to identify, based on the input, whether a heart rhythm is abnormal.

2.    The method according to claim 1, wherein the target feature further comprises a third feature and a fourth feature, the third feature is a modulus of an angle corresponding to the first straight line, and the fourth feature is a modulus of an angle corresponding to the second straight line.

3. The method according to claim 1 or 2, wherein the target feature further comprises a fifth feature and a sixth feature, the fifth feature is a standard deviation of the angle corresponding to the first straight line, and the sixth feature is a standard deviation of the angle corresponding to the second straight line.

4. The method according to any one of claims 1 to 3, wherein the target feature further comprises a seventh feature, an eighth feature, and a ninth feature, the seventh feature is a ratio of a quantity of data pieces of the heart rate data that are located in an area 0 in the Poincare plot to a total quantity of pieces of the heart rate data, the eighth feature is a standard deviation of an angle that uses heart rate data located in the area 2 as a vertex in three pieces of adjacent heart rate data of the heart rate data that are respectively located in an area 1, the area 2, and an area 3 in the Poincare plot, and the ninth feature is a standard deviation of an angle that uses heart rate data located in the area 4 as a vertex in three pieces of adjacent heart rate data of the heart rate data that are respectively located in an area 6, the area 4, and an area 5 in the Poincare plot.

5. The method according to any one of claims 1 to 4, wherein the heart rate data is obtained based on inter-beat interval IBI data of a photoplethysmography PPG signal, and the target feature further comprises at least one of the following: a ratio of a quantity of differences greater than 50 milliseconds between adjacent pieces of data in the IBI data to a total quantity of pieces in the IBI data, a mean value of the IBI data, a median of the IBI data, a standard deviation of the IBI data, a root mean square of differences between adjacent pieces of data in the IBI data, or a standard deviation of the differences between the adjacent pieces of data in the IBI data.

6. The method according to any one of claims 1 to 5, wherein the heart rate data is obtained based on the IBI data of the PPG signal; and
the method further comprises:

   generating a power spectrum chart based on the IBI data; and
   determining at least one of a ratio of low frequency power to high frequency power, ultra low frequency power, the low frequency power, or the high frequency power in the power spectrum chart as the target feature.

7. The method according to any one of claims 1 to 6, wherein the heart rate data is obtained based on the IBI data of the PPG signal; and
the method further comprises:

   determining a sample entropy and/or a Shannon entropy based on the IBI data; and
   determining the sample entropy and/or the Shannon entropy as the target feature.

8. The method according to any one of claims 1 to 7, wherein the obtaining heart rate data of a user comprises:

   collecting a PPG signal of the user;
   determining whether the user is in a static state when the PPG signal is collected; and
   determining the heart rate data based on IBI data in the PPG signal if the user is in the static state when the PPG signal is collected.

9. The method according to claim 8, wherein the method further comprises:

   if the user is not in the static state when the PPG signal is collected, determining whether a duration within which the user is not in the static state exceeds a first duration; and
   determining the heart rate data based on the IBI data in the PPG signal if the duration within which the user is not in the static state does not exceed the first duration.

10. The method according to any one of claims 1 to 7, wherein the obtaining heart rate data of a user comprises:

   collecting a PPG signal of the user; and
   determining the heart rate data based on IBI data in the PPG signal if a peak value of the PPG signal is greater than or equal to a preset peak value.

11. The method according to any one of claims 8 to 10, wherein the collecting a PPG signal of the user comprises: collecting a PPG signal of the user if the user keeps in the static state within a second duration.

12. The method according to any one of claims 1 to 11, wherein the identification model is a random forest, a gradient boosting decision tree, extreme gradient boosting, or a support vector machine.

13. The method according to any one of claims 1 to 12, wherein the identification result is a sinus rhythm, premature beat, or atrial fibrillation.

14. A wearable device, comprising: a processor and a memory, wherein

the memory stores computer-executable instructions; and
the processor executes the computer-executable instructions stored in the memory, to enable the wearable device to perform the method according to any one of claims 1 to 13.

15. A computer-readable storage medium, wherein the computer-readable storage medium stores a computer program, and when the computer program is executed by a processor, the method according to any one of claims 1 to 13 is implemented.

16. A computer program product, comprising a computer program, wherein when the computer program is run, a computer is enabled to perform the method according to any one of claims 1 to 13.

Wearable device

| PPG module [150] | Wireless communication module BT/NFC [160] |

Sensor module [180]

Pressure sensor [180A]

Gyroscope sensor [180B]

Barometric pressure sensor [180C]

Magnetic sensor [180D]

Acceleration sensor [180E]

Distance sensor [180F]

Proximity light sensor [180G]

Fingerprint sensor [180H]

Temperature sensor [180J]

Touch sensor [180K]

Ambient light sensor [180L]

Bone conduction sensor [180M]

Speaker [170A]

Receiver [170B]

Microphone [170C]

Audio module [170]

Display screens 1-N [193]

Indicator [192]

Button [190]

Internal memory [121]

External memory interface [120]

Processor [110]

USB interface [130]

Charging input

Charging management module [140]

Power management module [141]

FIG. 1

200

Obtain acceleration data by using an Acc, and collect a PPG signal — S201

Determine whether a wearable device is in a static state — S202

No →

Yes ↓

Preprocess the PPG signal to obtain a preprocessed PPG signal — S203

Whether a non-static duration exceeds a preset duration A — S207

No →

Yes ↓

Extract peak value data in the preprocessed PPG signal — S204

Exit heart rhythm abnormality identification — S208

Whether the peak value data exceeds a preset peak value — S205

No →

Yes ↓

Whether a collection duration exceeds a preset duration B — S206

No ←

Yes ↓

Obtain a peak value set based on peak value data within the collection duration — S209

Obtain an IBI set based on the peak value set — S210

Calculate a heart rate corresponding to each element in the IBI set, to obtain a heart rate set — S211

Calculate differences between adjacent heart rates based on the heart rate set, to obtain a difference set — S212

Construct coordinates based on adjacent elements in the difference set, to obtain a coordinate set — S213

Extract a statistical feature based on areas, in a Poincare plot, of elements in the coordinate set — S214

Input the statistical feature to a machine learning model, to obtain an identification result — S215

FIG. 2

FIG. 3

FIG. 4

FIG. 5

FIG. 6

700

```
┌─────────────────────────────────────────┐
│ Obtain acceleration data within 1 second by │  S701
│ using an Acc                             │
└─────────────────────────────────────────┘
                    │
                    ▼
┌─────────────────────────────────────────┐
│ Determine a motion state within the 1    │  S702
│ second based on the acceleration data within │
│ the 1 second                             │
└─────────────────────────────────────────┘
                    │
                    ▼
            ◇ Whether an              ◇  S703
      acceleration obtaining duration
No    is greater than or equal to 3
            seconds
                    │ Yes
                    ▼
            ◇ Whether a              ◇  S704
      static state is kept within 3
            seconds              No
        Yes │                          │
            ▼                          ▼
┌───────────────────────┐   ┌─────────────────────┐
│        S201           │   │ Exit heart rhythm    │  S705
│                       │   │ abnormality identification │
└───────────────────────┘   └─────────────────────┘
```

FIG. 7

```
┌─────────────────┐
│ PPG signals of the │
│ sinus rhythm     │──┐
└─────────────────┘  │
                     │   ┌──────────────┐   ┌──────────┐   ┌──────────┐   ┌──────────────────┐
┌─────────────────┐  │   │ Statistical features, │ │ Initial  │ │ Model    │ │     Label        │
│ PPG signals of the │──┼──▶│ time-domain  │──▶│ machine  │──▶│ output   │ │ ┌──────────────┐ │
│ atrial fibrillation │  │   │ features,    │   │ learning │   │ result   │ │ │ Sinus rhythm │ │
└─────────────────┘  │   │ frequency-domain │ │ model    │ │          │ │ └──────────────┘ │
                     │   │ features, and non- │ │          │ │          │ │ ┌──────────────┐ │
┌─────────────────┐  │   │ linear features of │ │          │ │          │ │ │ Atrial        │ │
│ PPG signals of the │──┘   │ the PPG signals │ │          │ │          │ │ │ fibrillation │ │
│ premature beat   │       └──────────────┘   └──────────┘   └──────────┘ │ └──────────────┘ │
└─────────────────┘                                                        │ ┌──────────────┐ │
                                                                           │ │ Premature     │ │
                                                                           │ │ beat          │ │
                                                                           │ └──────────────┘ │
                                                                           └──────────────────┘
                                   ┌──────────────┐
                                   │ Loss function │
                                   └──────────────┘
```

FIG. 8

PPG signal of the sinus rhythm

PPG signal of the atrial fibrillation

PPG signals of the premature beat

## FIG. 9

Poincare plot

Sinus rhythm

Poincare plot

Normal premature beat

Poincare plot

Normal atrial fibrillation

FIG. 10

Poincare plot

Premature beat with bigeminy

Poincare plot

Premature beat with trigeminy

FIG. 11

Wearable device 1200

Obtaining module 1210

Processing module 1220

FIG. 12

Wearable device 1300

Processor 1310

Memory 1330

Transceiver 1320

FIG. 13

## INTERNATIONAL SEARCH REPORT

| International application No. |
|---|
| **PCT/CN2023/120880** |

### A. CLASSIFICATION OF SUBJECT MATTER

A61B 5/024(2006.01)i

According to International Patent Classification (IPC) or to both national classification and IPC

### B. FIELDS SEARCHED

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT, WPABSC, ENTXTC, CJFD: 心律失常, 心律不齐, 窦性心律, 早搏, 房颤, 心率, 庞卡莱, 庞加莱, 潘凯, 图, 直线, 原点, 距离, 标准差, 机器学习, 模型, 识别, arrhythmia, sinus, rhythm, premature, beat, atrial, fibrillation, heart, rate, poincare, plot, straight, line, original, point, distance, standard, deviation, machine, learning, model, identification, recognition

### C. DOCUMENTS CONSIDERED TO BE RELEVANT

| Category* | Citation of document, with indication, where appropriate, of the relevant passages | Relevant to claim No. |
|---|---|---|
| PX | CN 116746900 A (HONOR TERMINAL CO., LTD.) 15 September 2023 (2023-09-15) claims 1-16 | 1-16 |
| A | CN 115120248 A (ZHEJIANG LAB) 30 September 2022 (2022-09-30) description, paragraphs [0003]-[0048], claims 1-10, and figures 1-8 | 1-16 |
| A | US 2014330134 A1 (WORCESTER POLYTECHNIC INSTITUTE) 06 November 2014 (2014-11-06) entire document | 1-16 |
| A | CN 106073755 A (CHENGDU XINHUI JUYUAN TECHNOLOGY CO., LTD.) 09 November 2016 (2016-11-09) entire document | 1-16 |
| A | CN 108403107 A (PEKING UNIVERSITY SHENZHEN GRADUATE SCHOOL) 17 August 2018 (2018-08-17) entire document | 1-16 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **15 December 2023** | **21 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 559 383 A1**

## INTERNATIONAL SEARCH REPORT

International application No.

**PCT/CN2023/120880**

### C. DOCUMENTS CONSIDERED TO BE RELEVANT

| Category* | Citation of document, with indication, where appropriate, of the relevant passages | Relevant to claim No. |
|---|---|---|
| A | CN 110090012 A (SHANGHAI TURING MEDICAL TECHNOLOGY CO., LTD.) 06 August 2019 (2019-08-06)<br>entire document | 1-16 |
| A | US 2022183608 A1 (SAMSUNG ELECTRONICS CO., LTD. et al.) 16 June 2022 (2022-06-16)<br>entire document | 1-16 |
| A | CN 114246569 A (HUAWEI TECHNOLOGIES CO., LTD.) 29 March 2022 (2022-03-29)<br>entire document | 1-16 |

Form PCT/ISA/210 (second sheet) (July 2022)

39

**INTERNATIONAL SEARCH REPORT**
Information on patent family members

International application No.

**PCT/CN2023/120880**

| Patent document cited in search report | | | Publication date (day/month/year) | Patent family member(s) | | | Publication date (day/month/year) |
|---|---|---|---|---|---|---|---|
| CN | 116746900 | A | 15 September 2023 | None | | | |
| CN | 115120248 | A | 30 September 2022 | None | | | |
| US | 2014330134 | A1 | 06 November 2014 | WO | 2014179544 | A1 | 06 November 2014 |
| | | | | US | 9408576 | B2 | 09 August 2016 |
| CN | 106073755 | A | 09 November 2016 | None | | | |
| CN | 108403107 | A | 17 August 2018 | None | | | |
| CN | 110090012 | A | 06 August 2019 | None | | | |
| US | 2022183608 | A1 | 16 June 2022 | US | 11564613 | B2 | 31 January 2023 |
| CN | 114246569 | A | 29 March 2022 | WO | 2022062992 | A1 | 31 March 2022 |

Form PCT/ISA/210 (patent family annex) (July 2022)

## EP 4 559 383 A1

**Patent documents cited in the description**

- CN 202211282519